(19) Europäisches Patentamt
European Patent Office
Office européen des brevets

(11) Numéro de publication : **0 053 962 B1**

(12) # FASCICULE DE BREVET EUROPÉEN

(45) Date de publication du fascicule du brevet : **13.03.85**

(51) Int. Cl.[4] : **C 07 D501/24**

(21) Numéro de dépôt : **81401825.5**

(22) Date de dépôt : **19.11.81**

(54) **Nouvelles céphalosporines et leur préparation.**

(30) Priorité : **20.11.80 FR 8024636**

(43) Date de publication de la demande : **16.06.82 Bulletin 82/24**

(45) Mention de la délivrance du brevet : **13.03.85 Bulletin 85/11**

(84) Etats contractants désignés : **AT BE CH DE FR GB IT LI LU NL SE**

(56) Documents cités : **FR-A- 2 102 080**

(73) Titulaire : **RHONE-POULENC SANTE**
**Les Miroirs 18 Avenue d'Alsace**
**F-92400 Courbevoie Cedex (FR)**

(72) Inventeur : **Farge, Daniel**
**30 rue des Pins Sylvestres**
**F-94320 Thiais (FR)**
Inventeur : **Le Roy, Pierre**
**2 Allée des Cerisiers**
**F-94320 Thiais (FR)**
Inventeur : **Moutonnier, Claude**
**3 rue Auguste Rodin**
**F-92350 Le Plessis-Robinson (FR)**
Inventeur : **Peyronel, Jean-François**
**36 parc d'Ardenay**
**F-91110 Palaiseau (FR)**

(74) Mandataire : **Gaumont, Robert et al**
**RHONE-POULENC RECHERCHES Service Brevets**
**Pharma 25, Quai Paul Doumer**
**F-92408 Courbevoie Cedex (FR)**

**EP 0 053 962 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouvelles formylméthyl-3 céphalosporines de formule générale

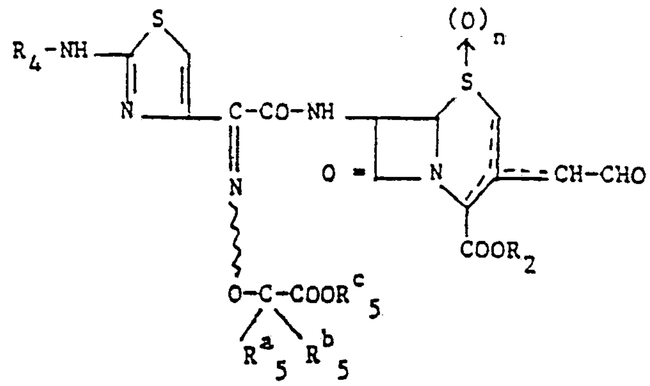

(I)

Dans la demande de brevet français publiée sous le numéro 2 321 294 ont été décrites des céphalosporines portant en position -3 un radical acylméthyle.

Les produits de formule générale (I), dans laquelle n est égal à 0 ou 1, se présentent sous forme oxoéthyl-3 bicyclooctène-2 ou -3 ou oxoéthylidène-3 bicyclooctane lorsque n = 0 et sous forme oxoéthyl-3 bicyclooctène-2 ou oxoéthylidène-3 bicyclooctane lorsque n = 1 (selon la nomenclature des Chemical Abstracts) et les radicaux $R^a_5$ et $R^b_5$, qui sont identiques ou différents, représentent des atomes d'hydrogène ou des radicaux alcoyle ou forment ensemble un radical alcoylène contenant 2 ou 3 atomes de carbone, $R^c_5$ représente un atome d'hydrogène ou un radical protecteur d'acide tel que méthoxyméthyle, t. butyle, benzhydryle, benzyle, nitrobenzyle ou p. méthoxybenzyle, $R_4$ représente un radical protecteur d'amine (tel que t. butoxycarbonyle, trichloro-2,2,2 éthoxycarbonyle, formyle, chloracétyle, trichloracétyle, trifluoracétyle, trityle, benzyle, dibenzyle, benzyloxycarbonyle, p. nitrobenzyloxycarbonyle ou p. méthoxybenzyloxycarbonyle) et $R_2$ représente un radical facilement éliminable par voie enzymatique, de formule générale

$$-\underset{\displaystyle R_9}{\underset{|}{CH}} - O - CO - R_8 \qquad \text{(II)}$$

(dans laquelle $R_8$ représente un radical alcoyle ou le radical cyclohexyle et $R_9$ représente un atome d'hydrogène ou un radical alcoyle) ou un radical protecteur d'acide tel que méthoxyméthyle, t. butyle, benzhydryle, benzyle, nitrobenzyle ou p. méthoxybenzyle.

Les portions ou radicaux alcoyles ou acyles cités ci-dessus (ou qui seront cités ci-après) sont (sauf mention spéciale) droits ou ramifiés et contiennent 1 à 4 atomes de carbone.

Les mélanges des isomères oxoéthyl-3 bicyclooctène-2 et -3 et oxoéthylidène-3 bicyclooctane entrent dans le cadre de l'invention.

Par ailleurs, il est entendu que le groupement carboxyalcoyloxy du radical acylamino-7 peut se trouver dans l'une des positions syn ou anti et que ces isomères et leurs mélanges entrent aussi dans le cadre de la présente invention.

La forme syn peut être représentée par la formule

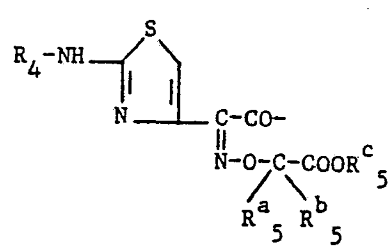

(IIIa)

La forme anti peut être représentée par la formule

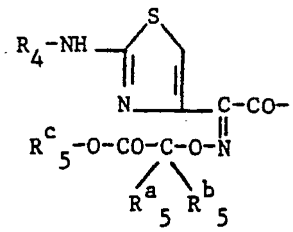

(IIIb)

De plus lorsque $R^a_5$ et $R^b_5$ sont différents il existe des diastéréoisomères.

Les mélanges d'isomères des produits définis ci-dessus entrent également dans le cadre de l'invention.

Selon l'invention, les produits de formule générale (I) pour lesquels n = 0 peuvent être obtenus par hydrolyse d'une énamine (ou du mélange des isomères) de formule générale

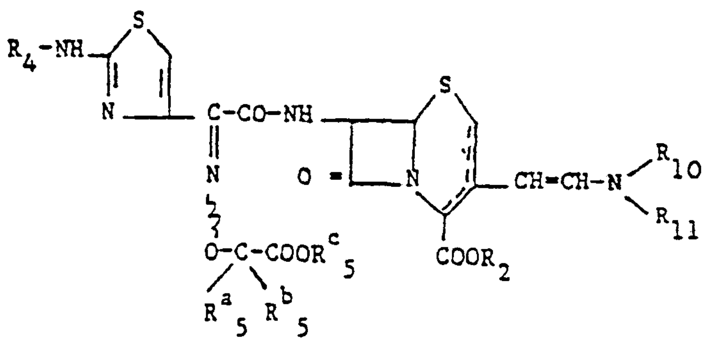

(IV)

dans laquelle $R_2$, $R_4$, $R^a_5$, $R^b_5$ et $R^c_5$ sont définis comme précédemment (à l'exception pour $R^c_5$ de représenter un atome d'hydrogène) et $R_{10}$ et $R_{11}$, qui sont identiques ou différents, représentent des radicaux alcoyle (éventuellement substitués par un radical hydroxy, alcoyloxy, amino, alcoylamino ou dialcoylamino) ou phényle, ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, étant entendu que l'énamine de formule générale (IV) se présente sous forme bicyclooctène-2 ou -3 et que le substituant sur l'atome de carbone en position -3 du bicyclooctène présente la stéréoisomérie cis (ou « Z ») ou trans (ou « E »)

puis éventuellement élimination du radical protecteur $R^c_5$.

De préférence on hydrolyse une énamine de formule générale (IV) dans laquelle $R_{10}$ et $R_{11}$ représentent un radical méthyle.

On opère généralement dans un acide organique (par exemple acide formique, acide acétique) ou minéral (par exemple acide chlorhydrique, acide sulfurique) en présence ou non d'un solvant, en milieu aqueux ou organique, à une température comprise entre − 20 °C et la température de reflux du mélange réactionnel, puis traite éventuellement par une base minérale (bicarbonate alcalin) ou organique (amine tertiaire ou pyridine).

Lorsque l'on opère en milieu organique, l'hydrolyse est réalisée par addition d'eau au mélange réactionnel ; lorsque l'on opère en présence d'un solvant, il n'est pas nécessaire que le solvant soit miscible à la phase aqueuse acide : le contact est alors réalisé par agitation vive.

Parmi les solvants utilisables peuvent être cités les solvants chlorés, l'acétate d'éthyle, le tétrahydrofuranne, l'acétonitrile, le diméthylformamide, les alcools.

Lorsque l'on veut obtenir un produit de formule générale (I) dans laquelle le radical $R^c_5$ représente un atome d'hydrogène, il est nécessaire d'utiliser une énamine de formule générale (IV) dans laquelle les radicaux protecteurs ($R^c_5$ et $R_2$) des fonctions acides sont différents et éliminables sélectivement.

L'élimination du radical protecteur $R^c_5$ s'effectue dans les conditions décrites ci-après pour la libération d'un carboxy protégé.

2. Selon l'invention les produits de formule générale (I), pour lesquels n = 1, peuvent être obtenus par oxydation des produits de formule générale (I) pour lesquels n est égal à 0 par toute méthode connue qui n'altère pas le reste de la molécule, notamment par application de la méthode décrite dans la demande de brevet allemand DE 2 637 176.

Les produits de formule générale (IV) pour lesquels $R_{10}$ et $R_{11}$ ont les définitions données précédemment (à l'exception de représenter alcoyle substitué par hydroxy, amino ou alcoylamino) peuvent être obtenus par action d'un produit éventuellement préparé in situ de formule générale

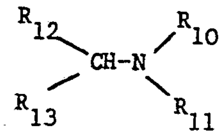

(V)

[[dans laquelle $R_{10}$ et $R_{11}$ sont définis comme précédemment et $R_{12}$ et $R_{13}$, qui sont identiques ou différents, soit représentent des groupements de formule générale

$$-X_2R_{14} \quad \text{(VI)}$$

(dans laquelle $X_2$ est un atome d'oxygène et $R_{14}$ représente un radical alcoyle ou phényle), soit représentent l'un un radical de formule générale (VI) dans laquelle $X_2$ est oxygène ou soufre et l'autre un radical amino de formule générale

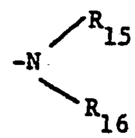

(VII)

[dans laquelle $R_{15}$ et $R_{16}$ sont définis comme $R_{10}$ et $R_{11}$ dans la formule générale (V)], soit encore représentent chacun un radical de formule générale (VII)]] sur un dérivé de céphalosporine de formule générale

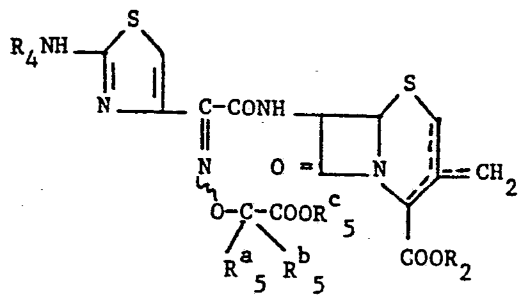

(VIII)

dans laquelle les différents symboles étant définis comme précédemment pour la formule générale (IV), le dérivé se présente sous forme méthyl-3 bicyclooctène-2 ou -3 ou méthylène-3 bicyclooctane.

On opère généralement dans un solvant organique tel que diméthylformamide, hexaméthylphosphorotriamide, diméthylacétamide, acétonitrile, acétate d'éthyle, dioxanne ou solvant chloré (par exemple dichloro-1,2 éthane) ou dans un mélange de tels solvants (diméthylformamide-tétrahydrofuranne, diméthylformamide-diméthylacétamide, diméthylformamide-éther ou diméthylformamide-dioxanne par exemple) à une température comprise entre 20 °C et la température de reflux du mélange réactionnel.

Lorsque l'on choisit un produit de formule générale (V) dans laquelle le radical (VII) est différent de $-NR_{10}R_{11}$, il est préférable de choisir un tel produit de manière que l'amine $HNR_{15}R_{16}$ soit plus volatile que $HNR_{10}R_{11}$.

Les produits de formule générale (IV) dans laquelle $R_{10}$ et $R_{11}$, qui sont identiques ou différents, représentent des radicaux alcoyle substitués par hydroxy, amino ou alcoylamino, peuvent être obtenus par transénamination à partir d'un produit de formule générale (IV) dans laquelle $R_{10}$ et $R_{11}$ représentent des radicaux alcoyle, de préférence méthyle.

La réaction s'effectue par action d'une amine de formule générale

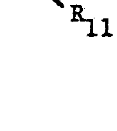

(IX)

(dans laquelle $R_{10}$ et $R_{11}$ ont les définitions correspondantes) sur le produit de formule générale (IV), et l'on opère dans des conditions analogues à celles décrites précédemment pour l'action d'un produit de formule générale (V) sur un dérivé de formule générale (VIII).

Les produits de formule générale (V) peuvent être préparés selon les méthodes décrites par H. BREDERECK et coll., Chem. Ber. *101*, 41 (1968), Chem. Ber. *101*, 3058 (1968) et Chem. Ber. *106*, 3725 (1973).

Les dérivés de la céphalosporine de formule générale (VIII) peuvent être préparés à partir des produits de formule générale

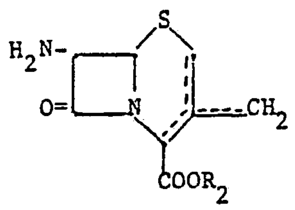

(X)

[dans laquelle $R_2$ est défini comme précédemment et la position de la double liaison est définie comme pour le produit de formule générale (VIII)] par action d'un acide de formule générale

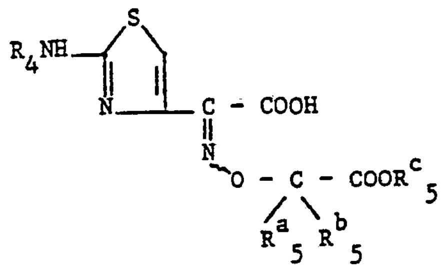

(XI)

dans laquelle $R_4$, $R^a_5$, $R^b_5$ et $R^c_5$ sont définis comme précédemment pour la formule générale (IV), ou d'un dérivé réactif de cet acide. Il est entendu que l'acide de formule générale (XI) sous forme syn, anti ou leurs mélanges, conduit respectivement aux produits de formule générale (VIII) de forme syn, anti ou à leurs mélanges.

Lorsque l'on utilise l'acide libre, on effectue généralement la condensation du produit de formule générale (XI) sur l'amino-7 céphalosporine de formule générale (X), dans un solvant organique tel que le diméthylformamide, l'acétonitrile, le tétrahydrofuranne, le chlorure de méthylène ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre − 20 et 40 °C.

Lorsque l'on utilise un dérivé réactif de l'acide de formule générale (XI), il est possible de mettre en œuvre l'anhydride, un anhydride mixte ou un ester réactif de formule générale

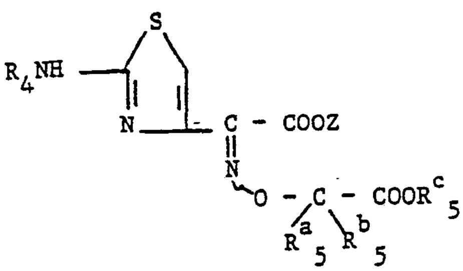

(XII)

[dans laquelle, $R_4$, $R^a_5$, $R^b_5$ et $R^c_5$ étant définis comme précédemment dans la formule générale (XI), Z représente un radical succinimido, benzotriazolyle-1, nitro-4 phényle, dinitro-2,4 phényle, pentachlorophényle ou phtalimido], ou bien des dérivés réactifs tels qu'un halogénure d'acide, par exemple le chlorure de l'acide de formule générale (XI) ou un thioloester.

Lorsque l'on met en œuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par

exemple l'acétone), ainsi que des mélanges des solvants ci-dessus, en présence d'un accepteur d'acide tel qu'un époxyde (par exemple l'oxyde de propylène) ou tel qu'une base organique azotée comme la pyridine, la diméthylaminopyridine, la N-méthylmorpholine, ou une trialcoylamine (par exemple triéthylamine), ou dans un milieu hydroorganique en présence d'un agent alcalin de condensation tel que le bicarbonate de sodium, et l'on opère à une température comprise entre − 40 et + 40 °C.

Il est également possible de mettre en œuvre une amino-7 céphalosporine de formule générale (X) préalablement silylée par application de la méthode décrite dans la demande de brevet allemand 2 804 040.

Lorsque l'on met en œuvre un ester réactif de formule générale (XII), on opère généralement en présence d'une trialcoylamine (par exemple triéthylamine) dans un solvant organique tel que le diméthylformamide, à une température comprise entre 0 et 40 °C.

Le dérivé de la céphalosporine de formule générale (VIII), dans laquelle $R_2$ représente un radical de formule générale (II), peut être obtenu par estérification de l'acide correspondant par toute méthode connue en soi pour préparer un ester à partir d'un acide, sans toucher au reste de la molécule.

Généralement on fait réagir un sel alcalin ou un sel d'amine tertiaire d'un produit de formule générale

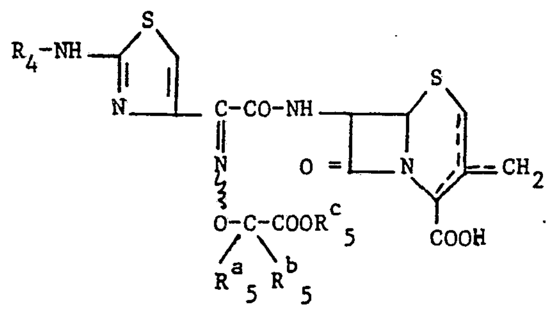

(XIII)

[dans laquelle $R_4$, $R^a_5$, $R^b_5$ sont définis comme précédemment, $R^c_5$ représente un radical protecteur et la position de la double liaison est définie comme pour le produit de formule générale (VIII)], sur un halogénure de formule générale

$$X - \underset{\displaystyle R_9}{\underset{|}{CH}} - OCO\ R_8 \qquad (XIV)$$

(dans laquelle $R_8$ et $R_9$ sont définis comme précédemment et X représente un atome d'halogène) dans un solvant inerte tel que le diméthylformamide à une température comprise entre 0 et 30 °C.

Le produit de formule générale (X) dans laquelle $R_2$ représente un radical de formule générale (II) peut être préparé de la même manière à partir du 7-ADCA.

Les produits de formule générale (XIV) peuvent être préparés selon la méthode décrite dans la demande de brevet allemand 2 350 230.

L'introduction du groupement protecteur $R_2$ dans le produit de formule générale (VIII) peut être effectuée sur une céphalosporine de formule générale (XIII) selon l'une des méthodes décrites dans les références suivantes :

— lorsque $R_2$ est méthoxyméthyle : selon S. Seki et coll., Tetrahedron Lett., *33*, 2915 (1977),

— lorsque $R_2$ est t. butyle : selon R. J. Stedman, J. Med. Chem., *9*, 444 (1966),

— lorsque $R_2$ est benzhydryle : selon la demande de brevet néerlandais 7 303 263,

— lorsque $R_2$ est benzyle, nitrobenzyle ou p. méthoxybenzyle : selon R. R. Chauvette et coll., J. Org. Chem., *38* (17), 2994 (1973).

Les acides de formule générale (XI) peuvent être préparés selon les méthodes décrites dans les brevets belges 864 810, 865 298, 876 541 et 876 542.

Les nouveaux produits de formule générale (I) sont utiles comme intermédiaires pour la préparation de thiovinyl-3 céphalosporines de formule générale :

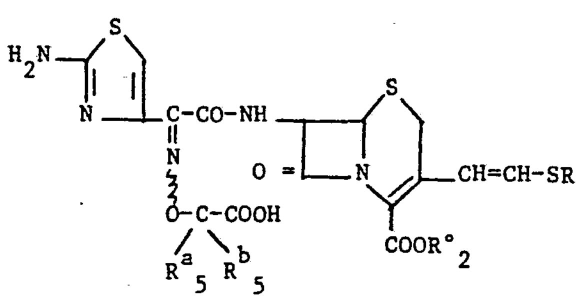

(XV)

dans laquelle le symbole R est choisi parmi les significations suivantes :

1) alcoyle, L-amino-2 carboxy-2 éthyle, phényle,

2) pyridyle-2, pyridyle-3 ou pyridyle-4 et leurs N-oxydes,

3) pyrimidinyle-2, pyridazinyle-3 substitué en position -6 (par un radical alcoyle, méthoxy, amino ou acylamino) et éventuellement N-oxydé ou tétrazolo[4,5-b] pyridazinyle-6,

4) dioxo-5,6 tétrahydro-1,4,5,6 triazine-1,2,4 yle-3 substitué en position -4, triazol-1,3,4 yle-5 ou alcoyloxycarbonyl-2 triazol-1,3,4 yle-5 substitués en position -1,

a) par un radical alcoyle contenant 1 à 4 atomes de carbone non substitué ou substitué par un radical alcoyloxy, alcoylthio, phényle, formyle, carbamoyle, alcoylcarbamoyle, dialcoylcarbamoyle, acyle, alcoyloxycarbonyle ou thiazolidinyle-2,

b) par un radical allyle, dihydroxy-2,3 propyle ; dihydroxy-1,3 propyle-2 ; formyl-2 hydroxy-2 éthyle ; formyloxy-3 hydroxy-2 propyle ; bis-formyloxy-2,3 propyle ou bis-formyloxy-1,3 propyle-2,

c) par un radical alcoyle contenant 2 à 4 atomes de carbone, substitué par hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales

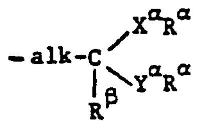

(XVIa)

ou

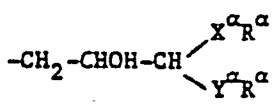

(XVIb)

ou

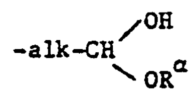

(XVIc)

dans laquelle alk est un radical alcoylène contenant 1 à 4 atomes de carbone, $X_\alpha$ et $Y_\alpha$ sont identiques et représentent des atomes d'oxygène ou de soufre et $R_\alpha$ représente un radical alcoyle, ou bien $X_\alpha$ et $Y_\alpha$ sont identiques ou différents et représentent des atomes d'oxygène ou de soufre et les radicaux $R\alpha$ forment ensemble un radical alcoylène contenant 2 ou 3 atomes de carbone, et $R^\beta$ représente un atome d'hydrogène ou un radical alcoyle contenant 1 à 3 atomes de carbone,

e) par un radical alcoyle contenant 2 à 5 atomes de carbone substitué par un radical alcoyloxyimino ou hydroxyimino.

5) dialcoyl-1,4 dioxo-5,6 tétrahydro-1,4,5,6 triazine-1,2,4 yle-3, alcoyl-1 dioxo-5,6 tétrahydro-1,4,5,6 triazine-1,2,4 yle-3 ou alcoyl-2 dioxo-5,6 tétrahydro-1,2,5,6 triazine-1,2,4 yle-3.

6) triazol-1,3,4 yle-5, triazol-1,2,3 yle-5 ou alcoyl-1 triazol-1,2,4 yle-5 non substitué ou substitué en position -3 par alcoyloxycarbonyle.

7) a) thiadiazol-1,3,4 yle-5 non substitué ou substitué par un radical alcoyle, trifluorométhyle, alcoyloxy, alcoylthio, hydroxyalcoylthio dont la partie alcoyle contient 2 à 4 atomes de carbone, alcoylsulfonyle, hydroxy, hydroxyalcoyle, carboxy, carboxyalcoyle, amino, alcoylamino, dialcoylamino, aminoalcoyle, alcoylaminoalcoyle, dialcoylaminoalcoyle, acylamino ou acylaminoalcoyle,

b) thiadiazol-1,2,4 yle-5 substitué par un radical alcoyle ou alcoyloxy,

8) a) oxadiazol-1,3,4 yle-5 non substitué ou substitué par un radical alcoyle, trifluorométhyle, phényle, aminoalcoyle, alcoylaminoalcoyle, dialcoylaminoalcoyle ou acylaminoalcoyle,

b) oxazolyle-2 ou alcoyl-4 oxazolyle-2,

9) tétrazolyle-5 non substitué ou substitué en position -1 par

a) un radical alcoyle contenant 1 à 4 atomes de carbone non substitué ou substitué par alcoyloxy, sulfo, carboxy, formyle ou sulfamoyle,

b) un radical alcoyle contenant 2 à 4 atomes de carbone substitué par hydroxy, amino, alcoylamino, dialcoylamino, acylamino, carboxyalcoylamino, sulfamoylamino, sulfoamino, uréido, alcoyluréido ou dialcoyluréido,

c) un radical alcoyle contenant 2 à 5 atomes de carbone substitué par hydroxyimino ou alcoyloxyimino,

d) un radical phényle ; dihydroxy-2,3 propyle ; dihydroxy-1,3 propyle-2 ; formyl-2 hydroxy-2 éthyle ; formyloxy-3 hydroxy-2 propyle ; bis-formyloxy-2,3 propyle ou bis-formyloxy-1,3 propyle-2, ou

e) un radical de formule générale (XVIa) pour lequel $R^\beta$ est un atome d'hydrogène, ou un radical de formule générale (XVIb),

10) dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2, triazol-1,2,4 yle-5 ou alcoyloxycarbonyl-3 triazol-1,2,4 yle-5 substitués en position -1 par

— un radical alcoyle lui-même substitué par un radical alcoyloxy, alcoylthio, phényle, formyle, carbamoyle, alcoylcarbamoyle, dialcoylcarbamoyle, hydroxyalcoylcarbamoyle (dont la partie alcoyle contient 2 à 4 atomes de carbone), acyle, alcoyloxycarbonyle ou thiazolidinyle-2

— un radical tel que défini précédemment en 4-b, c, d et e)

11) dioxo-5,6 hydroxyalcoylcarbamoylalcoyl-4 tétrahydro-1,4,5,6 triazine-1,2,4 yle-3 dont la partie hydroxyalcoyle contient 2 à 4 atomes de carbone,

12) alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1, par un radical formylalcoyle ou par un radical de formule générale (XVIa) dans laquelle Rβ est un atome d'hydrogène, les symboles $R^a_5$ et $R^b_5$ sont définis comme précédemment, et le symbole $R^o_2$ représente un atome d'hydrogène ou un radical de formule générale (II).

Les produits de formule générale (XV) définis ci-dessus présentent les mêmes isoméries que les produits de formule générale (I). De plus le substituant en position -3 du bicyclooctène présente la stéréoisomérie E ou Z.

Les produits de formule générale (XV) existent également à l'état de mélanges de ces isomères.

Les produits de formule générale (XV) peuvent être obtenus à partir des produits de formule générale (I) en opérant comme suit :

I/ Les thiovinyl-3 céphalosporines de formule générale (XV) dans laquelle R ne contient pas de substituant de formule générale (XVIc) peuvent être préparées de la manière suivante :

$A_1$/ On fait agir une forme activée d'un acide $R'_3SO_3H$ ou $R''_3COOH$, de formules générales

$(R'_3SO_2)_2O$ (XVIIa)

$R'_3SO_2Hal$ (XVIIb)

$(R''_3CO)_2O$ (XVIIc)

$R''_3COHal$ (XVIId)

(dans lesquelles $R'_3$ représente un radical alcoyle, trifluorométhyle, trichlorométhyle ou un radical phényle éventuellement substitué par un atome d'halogène ou par un radical alcoyle ou nitro, $R''_3$ est défini comme $R'_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle et Hal représente un atome d'halogène) ou un réactif d'halogénation, sur un produit de formule générale (I) ou sur un mélange de ses isomères, puis on réduit éventuellement le sulfoxyde obtenu et, le cas échéant, élimine les groupements protecteurs de la fonction amine et/ou éventuellement des fonctions acides pour obtenir un produit de formule générale

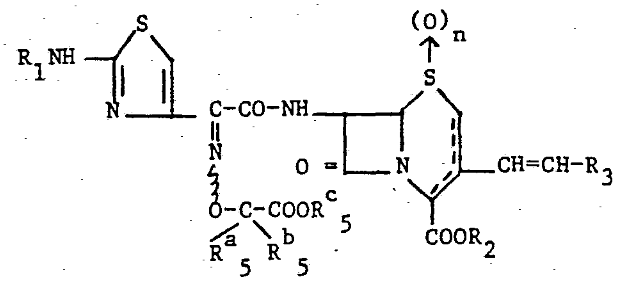

(XVIII)

[dans laquelle $R^a_5$, $R^b_5$ et $R^c_5$ sont définis comme précédemment pour la formule générale (I), $R_1$ est défini comme $R_4$ ou représente un atome d'hydrogène, $R_2$ est défini comme précédemment ou représente un atome d'hydrogène et $R_3$ représente un radical de formule générale

$R'_3—SO_2—O—$ (XIXa)

ou

$R''_3—COO—$ (XIXb)

(dans lesquelles $R'_3$ et $R''_3$ sont définis comme précédemment) ou un atome d'halogène choisi parmi le chlore, le brome et l'iode] qui se présente sous forme bicyclooctène-2 ou -3 lorsque n = 0 ou sous forme bicyclooctène-2 lorsque n = 1 et dont le substituant sur l'atome de carbone en position -3 du bicyclooctène présente la stéréoisomérie E ou Z.

Lorsque l'on veut préparer un produit de formule générale (XVIII) dans laquelle $R_3$ représente un atome d'halogène, on effectue la réaction à partir d'un produit de formule générale (I) dans laquelle $R^c_5$

est autre qu'un atome d'hydrogène.

On opère généralement en présence d'une base tertiaire de formule générale

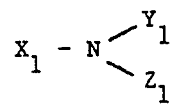

(XX)

[dans laquelle $X_1$, $Y_1$ et $Z_1$ représentent des radicaux alcoyle ou phényle, ou éventuellement deux d'entre eux forment un cycle avec l'atome d'azote auquel ils sont rattachés (par exemple en présence de triéthylamine ou de diméthylaniline)], dans un solvant organique chloré (par exemple dichlorométhane), dans un ester (par exemple acétate d'éthyle), dans un éther (par exemple dioxanne, tétrahydrofuranne), dans un amide (par exemple diméthylacétamide, diméthylformamide), dans l'acétonitrile ou la N-méthylpyrrolidone ou dans un mélange de ces solvants ou directement dans un solvant basique comme la pyridine, ou bien lorsque $R_3$ est autre qu'un atome d'halogène on opère en milieu hydroorganique en présence d'un agent alcalin de condensation (par exemple bicarbonate alcalin, soude ou potasse), à une température comprise entre − 78 °C et la température de reflux du mélange réactionnel.

Eventuellement on opère sous azote.

Il n'est pas absolument nécessaire d'avoir purifié préalablement le produit de formule générale (I) (ou le mélange d'isomères) pour le mettre en œuvre dans cette réaction.

Lorsque l'on veut préparer un produit de formule générale (XVIII) dans laquelle $R_3$ est un atome d'halogène, les agents d'halogénation peuvent être choisis parmi des dérivés halogénés du phosphore, notamment les

— composés d'addition d'halogène et de triarylphosphite, ou bien

— le trichlorure de phosphore, l'oxychlorure de phosphore, le pentachlorure de phosphore, le dichlorotriphénylphosphorane ou le catéchyltrichlorophosphorane lorsque $R_3$ est un atome de chlore, ou

— le tribromure de phosphore, l'oxybromure de phosphore, le pentabromure de phosphore ou le catéchyltribromophosphorane lorsque $R_3$ est un atome de brome.

Lorsque l'on utilise le trichlorure (ou le tribromure) de phosphore, on fait agir ce réactif sur un produit de formule générale (I) dans laquelle n = 0.

Le catéchyltrichloro (ou tribromo) phosphorane, qui peut être préparé in situ, peut être obtenu selon la méthode décrite par H. GROSS et U. KARSH, J. Prakt. Chem., *29*, 315 (1965).

Les composés d'addition d'halogène et de triarylphosphite, qui peuvent être formés in situ, sont décrits par H. N. RYDON et B. L. TONGE, J. Chem. Soc., 3043 (1956), par J. MICHALSKI et coll., J. Org. Chem., *45*, 3122 (1980) ou dans le brevet belge 881 424 et peuvent être préparés selon les méthodes mentionnées dans ces documents.

La préparation des dérivés halogénés de formule générale (XVIII) s'effectue en milieu anhydre.

Lorsque l'on veut préparer un produit de formule générale (XVIII) dans laquelle $R_3$ est un atome de chlore ou de brome, selon les conditions opératoires on peut isoler l'intermédiaire dihalogéné de formule générale

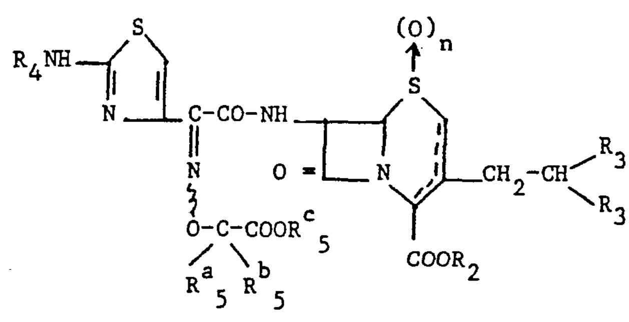

(XVIIIa)

[dans laquelle $R_2$, $R_4$, $R^a_5$, $R^b_5$ et $R^c_5$ sont définis comme pour les produits de formule générale (I) à l'exception pour $R^c_5$ de représenter un atome d'hydrogène, $R_3$ est défini comme ci-dessus, et qui se présente sous forme bicyclooctène-2 ou -3 lorsque n = 0 et sous forme bicyclooctène-2 lorsque n = 1] qui est alors déhydrohalogéné.

Lorsque l'on veut isoler l'intermédiaire dihalogéné, on opère par action d'un agent d'halogénation, dans un solvant organique tel qu'un solvant chloré (par exemple dichlorométhane, chloroforme, tétrachlorure de carbone, dichloro-1,2 éthane), un éther (par exemple éther éthylique, oxyde de propylène, tétrahydrofuranne, dioxanne), un amide (par exemple diméthylacétamide, diméthylpropionamide, diméthylformamide, N-acétylmorpholine, N-acétylpipéridine ou N-méthylpyrrolidone) ou un mélange de tels solvants à une température un peu moins élevée que pour préparer le dérivé halogénovinyl correspondant, c'est-à-dire comprise entre − 78 et 30 °C. Il est également possible

d'opérer en présence d'une base telle que la pyridine dans l'un des solvants ci-dessus, à une température comprise entre − 78 et 0 °C.

La déhydrohalogénation s'effectue en présence d'une base tertiaire telle que définie précédemment, d'une amine aromatique (par exemple pyridine, picoline, quinoléine) ou d'une base minérale (telle que la soude, la potasse, un carbonate ou un bicarbonate alcalin ou alcalino-terreux), en milieu organique ou hydroorganique dans les solvants cités précédemment, à une température comprise entre − 20 °C et la température de reflux du mélange réactionnel.

Il n'est pas absolument nécessaire d'avoir purifié l'intermédiaire dihalogéné pour procéder à sa déhydrohalogénation.

La réduction du S-oxyde peut être effectuée dans les conditions décrites dans la demande de brevet allemand 2 637 176.

Le cas échéant, l'élimination des radicaux protecteurs de la fonction amine et des fonctions acides s'effectue simultanément ou successivement.

A titre d'exemple :

1. L'élimination des groupements protecteurs d'amines s'effectue :

— lorsqu'il s'agit d'un radical t. butoxycarbonyle, trityle, p. méthoxybenzyloxycarbonyle ou formyle : par traitement en milieu acide. De préférence on utilise l'acide trifluoracétique en opérant à une température comprise entre 0 et 20 °C, ou bien on utilise l'acide formique, phosphorique ou polyphosphorique purs ou en présence d'eau, à température comprise entre 20 et 60 °C, ou encore l'acide paratoluènesulfonique ou méthanesulfonique dans l'acétone ou l'acétonitrile à une température comprise entre 20 °C et la température de reflux du mélange réactionnel. Dans ces conditions le produit de formule générale (I) peut être obtenu sous forme de trifluoroacétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique.

— lorsqu'il s'agit d'un radical trichloro-2,2,2 éthoxycarbonyle ou p-nitrobenzyloxycarbonyle : par réduction (notamment traitement par le zinc dans l'acide acétique),

— lorsqu'il s'agit d'un radical chloracétyle ou trichloracétyle : par application de la méthode décrite dans le brevet français publié sous le n° 2 243 199,

— lorsqu'il s'agit d'un radical benzyle, dibenzyle ou benzyloxycarbonyle : par hydrogénation catalytique,

— lorsqu'il s'agit d'un radical trifluoroacétyle : par traitement en milieu basique.

2. L'élimination des groupements protecteurs du radical carboxy s'effectue :

— lorsqu'il s'agit d'un groupement t. butyle, p. méthoxybenzyle ou benzyhydryle : par traitement en milieu acide, dans les conditions décrites ci-dessus pour l'élimination du radical trityle protecteur d'amino. Dans le cas du radical benzhydryle, on peut opérer en présence d'anisole

— lorsqu'il s'agit d'un groupement méthoxyméthyle : par traitement en milieu acide dilué

— lorsqu'il s'agit d'un groupement nitrobenzyle : par réduction (notamment traitement par le zinc dans l'acide acétique ou hydrogénolyse).

$B_1$/ Eventuellement, on oxyde un produit de formule générale (XVIII) dans laquelle n = 0 pour obtenir un produit de formule générale (XVIII) dans laquelle n = 1.

L'oxydation peut être effectuée par toute méthode connue qui n'altère pas le reste de la molécule, notamment par application de la méthode décrite dans le brevet allemand DE 2 637 176.

Il est également possible de préparer un produit de formule générale (XVIIIa) dans laquelle n = 1 à partir d'un produit de formule générale (XVIIIa) dans laquelle n = 0, en opérant comme ci-dessus.

$C_1$/ Eventuellement on estérifie un produit de formule générale (XVIII) dans laquelle $R_2$ représente un atome d'hydrogène, par toute méthode connue pour préparer un ester à partir d'un acide sans toucher au reste de la molécule, pour obtenir un produit de formule générale (XVIII) dans laquelle $R_2$ représente un radical de formule générale (II).

On opère notamment par action d'un sel alcalin ou d'un sel d'amine tertiaire du produit de formule générale (XVIII) sur un halogénure de formule générale (XIV), dans les conditions décrites précédemment pour la préparation des produits de formule générale (VIII) dans lesquels $R_2$ représente un radical de formule générale (II).

$D_1$/ Les thiovinyl-3 céphalosporines de formule générale (XV) dans laquelle R est défini comme précédemment, à l'exception de contenir un substituant de formule générale (XVIc), peuvent être préparées par action d'un thiol (libre ou sous forme de sel alcalin ou alcalino-terreux) de formule générale :

$$R{-}SH \qquad (XXI)$$

[dans laquelle le radical R, qui est défini comme ci-dessus, est protégé à l'état d'acétal tel que défini par les formules générales (XVIa) et (XVIb) lorsque l'on veut obtenir une céphalosporine de formule générale (XV) dans laquelle R contient un radical formyle ou acylalcoyle], sur un dérivé de la céphalosporine ou un mélange des isomères de formule générale (XVIII), suivie éventuellement de la réduction de l'oxyde

obtenu et de l'élimination des radicaux protecteurs.

Il est entendu que, lorsque le radical R du produit de formule générale (XXI) contient un groupement susceptible d'interférer avec la réaction, il est préférable de protéger ce groupement, par toute méthode connue en soi et qui n'altère pas le reste de la molécule (notamment lorsque R contient un radical amino, alcoylamino, hydroxy ou carboxy).

Lorsqu'il s'agit des groupements amino, alcoylamino ou carboxy, la protection s'effectue dans les conditions décrites précédemment.

Lorsqu'il s'agit de groupements hydroxy, la protection s'effectue par les radicaux tels que trityle, tétrahydropyrannyle ou méthoxy-2 propyle-2, ou sous forme d'acétal cyclique pour la protection des radicaux dihydroxy-2,3 propyle ou dihydroxy-1,3 propyle-2 (par exemple sous forme de radicaux diméthyl-2,2 dioxolannyl-4 méthyle ou diméthyl-2,2 dioxannyle-5).

Par ailleurs il est entendu que, lorsque le radical R du produit de formule générale (XXI) comporte un radical hydroxy ou sulfa il est préférable de mettre en œuvre un produit de formule générale (XVIIa) dans laquelle n = 0.

On opère généralement en présence d'une base telle qu'une pyridine ou une base organique tertiaire de formule générale (XX) on utilise par exemple la diisopropyléthylamine ou la diéthylphénylamine.

La présence d'une telle base n'est pas nécessaire lorsque l'on fait agir un sel alcalin ou alcalinoterreux du thiol de formule générale (XXI).

La réaction s'effectue avantageusement dans un solvant organique, tel que diméthylformamide, tétrahydrofuranne, méthanol, éthanol ou acétonitrile ou un mélange de tels solvants.

Il est également possible d'opérer en présence de bicarbonate alcalin dans un solvant tel que cité ci-dessus, éventuellement en présence d'eau.

On opère à une température comprise entre − 20 °C et la température de reflux du mélange réactionnel, la température choisie étant variable selon le thiol employé. De même, selon le thiol employé le temps de réaction peut varier de 5 minutes à 48 heures.

Eventuellement on opère sous azote.

De préférence lorsque l'on veut utiliser un bicyclooctène-3 de formule générale (I) on met en œuvre un tel produit pour lequel $R_2$ est autre que l'hydrogène.

L'élimination du radical protecteur de R peut être effectuée indifféremment avant ou après la réduction de l'oxyde, avant, simultanément ou après l'élimination des autres radicaux protecteurs.

La réduction de l'oxyde et l'élimination des groupements protecteurs d'amine et d'acide s'effectuent selon les méthodes décrites précédemment.

L'élimination des radicaux protecteurs de groupement hydroxy s'effectue :

— par acidolyse par exemple par l'acide trifluoroacétique, l'acide formique aqueux ou non ou l'acide paratoluènesulfonique lorsqu'il s'agit du radical trityle, tétrahydropyrannyle, diméthyl-2,2 dioxolannyl-4 méthyle ou diméthyl-2,2 dioxannyle. [Lorsque l'on utilise l'acide formique aqueux ou non, la libération des radicaux hydroxy protégés peut conduire au moins partiellement aux esters formiques correspondants, qui peuvent être séparés le cas échéant par chromatographie, notamment dans le cas des radicaux dihydroxypropyle protégés à l'état d'acétals cycliques dont le déblocage peut conduire au moins partiellement au mono ou diester formique correspondant],

— selon la méthode décrite dans le brevet belge 875 379 lorsqu'il s'agit du radical méthoxy-2 propyle-2.

L'élimination des groupements protecteurs dans les radicaux de formule générale (XVIa) et (XVIb) (lorsque l'on veut obtenir un produit de formule générale (XV) dans laquelle R contient un radical formyle ou acylalcoyle) s'effectue :

— en présence d'un acide sulfonique (par exemple acide méthanesulfonique ou acide p. toluènesulfonique) dans un solvant organique (par exemple acétonitrile ou acétone, éventuellement en présence d'eau et éventuellement en présence d'un réactif acétalisable tel que l'acétone, l'acide glyoxylique, le benzaldéhyde ou l'acide pyruvique, à une température comprise entre 20 °C et la température de reflux du mélange réactionnel

— ou bien, lorsque le radical R est un radical dioxo-5,6 tétrahydro-1,4,5,6 triazine-1,2,4 yle-3 : par action d'acide formique pur ou aqueux (contenant de préférence moins de 10 % d'eau), soit en présence ou non de silice, soit par transacétalisation en présence d'un réactif acétalisable tel que défini ci-dessus.

Les thiols de formule générale (XXI), qui peuvent être mis en œuvre sous leur forme tautomère, peuvent être préparés par application de l'une des méthodes suivantes selon la signification du radical R :

— lorsque R est un radical pyridyle-3 : selon la méthode décrite par H. M. Wuest et E. H. Sakal, J. Am. Chem. Soc., *73*, 1210 (1951),

— lorsque R est un radical oxyde-1 pyridyle-3 : selon la méthode décrite par B. Blank et coll., J. Med. Chem. *17*, 1065 (1974),

— lorsque R est un radical oxyde-1 pyridyle-4 : selon la méthode décrite par R. A. Y. Jones et coll., J. Chem. Soc. 2937 (1960),

— lorsque R est un radical pyridazinyle-3 substitué par alcoyle ou méthoxy et éventuellement N-oxydé : selon la méthode décrite dans le brevet belge 787 635,

— lorsque R est un radical pyridazinyle-3 substitué par amino et éventuellement N-oxydé : selon la méthode décrite dans le brevet belge 579 291,

— lorsque R est un radical pyridazinyle-3 substitué par acylamino et éventuellement N-oxydé : par application des méthodes décrites par M. Kumagai et M. Bando, Nippon Kagaku Zasshi, *84*, 995 (1963) et par T. Horie et T. Ueda, Chem. Pharm. Bull., *11*, 114 (1963),

— lorsque R est un radical tétrazolo [4,5-b] pyridazinyle-6 : selon la méthode décrite dans le brevet belge 804 251,

— lorsque R est un radical dioxo-5,6 tétrahydro-1,4,5,6 triazine-1,2,4 yle-3 substitué en position -4 ou alcoyloxycarbonyl-2 triazol-1,3,4 yle-5 substitué en position -1 par un radical $R^γ$ choisi parmi :

a) un radical allyle, alcoyle (1 à 4 atomes de carbone, lui-même éventuellement substitué par un radical alcoyloxy, alcoylthio, phényle, carbamoyle, alcoylcarbamoyle, dialcoylcarbamoyle, acyle, alcoyloxycarbonyle ou thiazolidinyle-2)

b) un radical dihydroxy-2,3 propyle ou dihydroxy-1,3 propyle-2 (éventuellement protégé sous forme d'acétal cyclique)

c) un radical alcoyle [2 à 4 atomes de carbone, lui-même substitué par hydroxy, carbamoyloxy, dialcoylamino, alcoylsulfinyle, alcoylsulfonyle, alcoylsulfonylamino, sulfamoylamino, acylamino (éventuellement substitué), alcoyloxycarbonylamino, uréido, alcoyluréido, dialcoyluréido],

d) un radical de formule générale (XVIa) ou (XVIb),

e) un radical hydroxyiminoalcoyle ou alcoyloxyiminoalcoyle ;

ou lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou alcoyloxycarbonyl-3 triazol-1,2,4 yle-5 substitué en position -1 par un radical $R^γ$ tel que défini ci-dessus (à l'exception de représenter un radical alcoyle non substitué) ou par un radical hydroxyalcoylcarbamoylalcoyle dont la portion hydroxyalcoyle contient 2 à 4 atomes de carbone, ou encore lorsque R est un radical dioxo-5,6 hydroxyalcoylcarbamoylalcoyl-4 tétrahydro-1,4,5,6 triazine-1,2,4 yle-3 dont la portion hydroxyalcoyle contient 2 à 4 atomes de carbone :

en faisant agir un oxalate d'alcoyle ou un halogénure de monooxalate d'alcoyle sur une thiosemicarbazide de formule générale

$$H_2N-CS-NH-NH-R^{γ} \qquad H_2N-CS-\underset{}{\overset{R^{γ}}{N}}-NH_2 \qquad \text{ou} \qquad R^{γ1}-NH-CS-NH-NH_2$$

(XXIIa) (XXIIb) (XXIIc)

(dans lesquelles $R^γ$ est défini comme ci-dessus et $R^{γ1}$ est défini comme $R^γ$ ou représente un radical hydroxyalcoylcarbamoylalcoyle).

On effectue la réaction en présence d'un alcoolate alcalin, par exemple l'éthylate ou le méthylate de sodium, ou le t. butylate de potassium, par application de la méthode décrite par M. Pesson et M. Antoine, Bull. Soc. Chim. France 1590 (1970).

Il n'est pas absolument nécessaire de purifier le produit obtenu (ni de libérer les radicaux protégés) pour le mettre en œuvre pour la préparation des produits de formule générale (XV).

Les thiosemicarbazides de formules générales (XXIIa) à (XXIIc) peuvent être préparées selon l'une des méthodes décrites par K. A. Jensen et coll., Acta Chem. Scand *22*, 1 (1968), ou par application de la méthode décrite par Y. Kazakov et J. Y. Potovskii, Doklady Acad. Nauk. SSSR *134*, 824 (1960), étant entendu que, lorsque $R^γ$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t. butoxycarbonyle, qui peut être éliminé par hydrolyse acide.

— Lorsque R est un radical triazol-1,3,4 yle-5 substitué en position -1

par un radical alcoyle, allyle ou alcoyloxyalcoyle,

par un radical alcoyle (1 à 4 atomes de carbone) lui-même substitué comme défini ci-dessus en a) à l'exception d'un radical thiazolidinyle-2,

par un radical tel que défini ci-dessus en c), ou

par un radical alcoyloxyiminoalcoyle :

par application de l'une des méthodes décrites par M. Pesson et M. Antoine, Bull. Soc. Chim. France 1590 (1970) ;

— Lorsque R est un radical triazol-1,3,4 yle-5 substitué en position -1 par thiazolidinyl-2 alcoyle ou hydroxyiminoalcoyle : par action respectivement de cystéamine ou d'hydroxylamine sur un dialcoyloxyalcoyl-1 mercapto-5 triazole-1,3,4 qui peut être obtenu par application de la méthode décrite par M. Kanaoka, J. Pharm. Soc. Japan, *75*, 1149 (1955), à partir d'une dialcoyloxyalcoyl-4 thiosemicarbazide.

— Lorsque R est un radical triazol-1,3,4 yle-5 substitué en position -1 par hydroxy-2,3 propyle ou dihydroxy-1,3 propyle-2 (éventuellement protégés sous forme d'acétal cyclique), ou représente un radical de formule générale (XVIa) ou (XVIb) : par application de la méthode décrite par M. Kanaoka, J. Pharm. Soc. Japan, *75*, 1149 (1955).

— Lorsque R est un radical dioxo-5,6 tétrahydro-1,4,5,6 triazine-1,2,4 yle-3 substitué en position -4 ou un radical alcoyloxycarbonyl-2 triazol-1,3,4 yle-5 ou triazol-1,3,4 yle-5 substitués en position -1 par acyloxyalcoyle (éventuellement substitué) : par acylation respectivement de la dioxo-5,6 hydroxyalcoyl-4 mercapto-3 tétrahydro-1,4,5,6 triazine-1,2,4, de l'alcoyloxycarbonyl-2 hydroxyalcoyl-1 mercapto-5 triazole-1,3,4 ou de l'hydroxyalcoyl-1 mercapto-5 triazole-1,3,4 dont le radical mercapto a été préalablement protégé [par exemple selon C. G. Kruse et coll., Tet. Lett. 1725 (1976)], par toute méthode connue pour acyler un alcool sans toucher au reste de la molécule, puis libération du groupement mercapto en milieu acide.

— Lorsque R est un radical dioxo-5,6 tétrahydro-1,4,5,6 triazine-1,2,4 yle-3 substitué en position -4 ou alcoyloxycarbonyl-2 triazol-1,3,4 yle-5 ou triazol-1,3,4 yle-5 substitués en position -1 par aminoalcoyle ou alcoylaminoalcoyle : par libération de la fonction amine du produit correspondant, protégé par exemple par un groupement t. butoxycarbonyle.

— Lorsque R est un radical dioxo-5,6 tétrahydro-1,4,5,6 triazine-1,2,4 yle-3 substitué en position -4, alcoyloxycarbonyl-2 triazol-1,3,4 yle-5 ou triazol-1,3,4 yle-5 substitués en position -1 par sulfoaminoalcoyle : à partir du produit correspondant substitué par un radical t. butoxycarbonylaminoalcoyle, par analogie avec la méthode décrite dans le brevet belge 847 237.

— Lorsque R est un radical dialcoyl-1,4 dioxo-5,6 tétrahydro-1,4,5,6 triazine-1,2,4 yle-3 ou alcoyl-1 dioxo-5,6 tétrahydro-1,4,5,6 triazine-1,2,4 yle-3 : selon la méthode décrite dans le brevet belge 830 455.

— Lorsque R est un radical alcoyl-2 dioxo-5,6 tétrahydro-1,2,5,6 triazine-1,2,4 yle-3 ou alcoyl-1 alcoyloxycarbonyl-3 triazol-1,2,4 yle-5 : selon la méthode décrite par M. Pesson et M. Antoine, C. R. Acad. Sci., Ser C, 267, 25, 1726 (1968).

— Lorsque R est un radical triazol-1,2,3 yle-5 : selon la méthode décrite dans la demande de brevet français 2 215 942.

— Lorsque R est un radical triazol-1,3,4 yle-5 : selon la méthode décrite par M. Kanaoka, J. Pharm. Soc. Jap. *75*, 1149 (1955).

— Lorsque R est un radical thiadiazol-1,3,4 yle-5 éventuellement substitué par alcoyle, alcoyloxy, alcoylthio, alcoylsulfonyle, amino, alcoylamino, dialcoylamino ou acylamino : selon les méthodes décrites dans le brevet belge 830 821.

— Lorsque R est un radical thiadiazol-1,3,4 yle-5 substitué par hydroxyalcoyle, aminoalcoyle, alcoylaminoalcoyle ou dialcoylaminoalcoyle : selon la méthode décrite dans la demande de brevet allemand 2 446 254.

— Lorsque R est un radical thiadiazol-1,3,4 yle-5 substitué par un radical carboxyalcoyle : par application de la méthode décrite dans la demande de brevet allemand 1 953 861.

— Lorsque R est un radical thiadiazol-1,3,4 yle-5 substitué par un radical trifluorométhyle : selon la méthode décrite dans la demande de brevet allemand 2 162 575.

— Lorsque R est un radical thiadiazol-1,3,4 yle-5 substitué par un radical carboxy : selon la méthode décrite dans la demande de brevet japonais 77 48666.

— Lorsque R est un radical thiadiazol-1,3,4 yle-5 substitué par un radical acylaminoalcoyle : selon la méthode décrite dans la demande de brevet japonais 76 80857.

— Lorsque R est un radical thiadiazol-1,3,4 yle-5 substitué par un radical hydroxyalcoylthio : par application de la méthode décrite par G. Nannini, Arz. Forsch. *27* (2), 343 (1977).

— Lorsque R est un radical thiadiazol-1,2,4 yle-5 substitué par alcoyle ou alcoyloxy : selon la méthode décrite dans la demande de brevet allemand 2 806 226 ou selon Chem. Ber. *90*, 184 (1957).

— Lorsque R est un radical oxadiazol-1,3,4 yle-5 tel que défini précédemment en 8a/ : par application de la méthode décrite par E. Hoggarth, J. Chem. Soc. 4811 (1952).

— Lorsque R est un radical oxazolyle-2 ou alcoyl-4 oxazolyle-2 : par application de la méthode décrite précédemment par C. Bradsher, J. Org. Chem. *32*, 2079 (1967).

— Lorsque R est un radical tétrazolyle-5 éventuellement substitué en position -1 par alcoyle, hydroxyalcoyle ou phényle : selon les méthodes décrites dans le brevet belge 830 821.

— Lorsque R est un radical tétrazolyle-5 substitué en position -1 par alcoyloxyalcoyle : par addition d'azoture de sodium sur un isothiocyanatoalcoyloxyalcoyle en opérant dans un solvant organique tel que l'éthanol, à la température de reflux du mélange réactionnel.

L'isothiocyanatoalcoyloxyaloyle peut être obtenu par application de la méthode décrite par E. Schmidt et coll., Chem. Ber. *73*, 286 (1940).

— Lorsque R est un radical tétrazolyle-5 substitué en position -1 par un radical carboxyalcoyle : selon la méthode décrite dans le brevet belge 858 112.

— Lorsque R est un radical tétrazolyle-5 substitué en position -1 par un radical sulfoalcoyle : selon la méthode décrite dans le brevet belge 856 498 ou décrite par D. A. Berges et coll., J. Het. Chem. *15*, 981 (1978).

— Lorsque R est un radical tétrazolyle-5 substitué en position -1 par un radical aminoalcoyle, alcoylaminoalcoyle, dialcoylaminoalcoyle : par application de la méthode décrite dans la demande de brevet allemand 2 738 711.

— Lorsque R est un radical tétrazolyle-5 substitué en position -1 par un radical sulfamoylalcoyle, sulfamoylaminoalcoyle ou sulfoaminoalcoyle : selon la méthode décrite dans le brevet belge 856 636.

— Lorsque R est un radical tétrazolyle-5 substitué par un radical acylaminoalcoyle ou thiadiazol-1,3,4 yle-5 substitué par hydroxy : selon la méthode décrite dans le brevet US 4 117 123.

— Lorsque R est un radical tétrazolyle-5 substitué en position -1 par un radical uréidoalcoyle, alcoyluréidoalcoyle ou dialcoyluréidoalcoyle : à partir du produit correspondant substitué par aminoalcoyle (dont le radical mercapto a été préalablement protégé), par traitement par un isocyanate alcalin, par un isocyanate d'alcoyle ou par un halogénure de dialcoylcarbamoyle, puis libération du groupement mercapto dans les conditions décrites dans le brevet belge 847 237.

— Lorsque R est un radical tétrazolyle-5 substitué en position -1 par un radical carboxyalcoylaminoalcoyle : selon la méthode décrite dans la demande de brevet allemand 2 715 597.

— Lorsque R est un radical tétrazolyle-5 substitué en position -1 par un radical dihydroxy-2,3 propyle : selon la méthode décrite dans le brevet US 4 064 242.

— Lorsque R est un radical tétrazolyle-5 substitué en position -1 par un radical dihydroxy-1,3 propyle-2 : par addition d'azoture de sodium sur un isothiocyanate de diméthyl-2,2 dioxolanne-1,3 yle-5 (suivie éventuellement de la libération des groupements hydroxy).

— Lorsque R est un radical tétrazolyle-5 substitué en position -1 par un radical de formule générale (XVIa) ou (XVIb) tels que définis précédemment en 9d/ ou un radical défini précédemment en 9e/ : par action d'azoture de sodium sur l'isothiocyanate correspondant, par analogie avec la méthode décrite R. E. Orth, J. Pharm. Sci. *52* (9), 909 (1963), étant entendu que dans le cas où R contient un substituant hydroxy ou hydroxyiminoalcoyle, l'alcool ou l'oxime sont éventuellement protégés par exemple par un groupement tétrahydropyrannyle.

— Lorsque R est un radical triazol-1,2,4 yle-5 substitué en position -1 par un radical allyle ou alcoyloxyalcoyle, par un radical alcoyle (1 à 4 atomes de carbone) lui-même substitué comme défini ci-dessus en a) à l'exception d'un radical thiazolidinyle-2, par un radical hydroxyalcoylcarbamoylalcoyle dont la portion hydroxyalcoyle contient 2 à 4 atomes de carbone, par un radical tel que défini ci-dessus en c), ou par un radical alcoyloxyiminoalcoyle : par analogie avec les méthodes décrites par M. Pesson et M. Antoine, Bull. Soc. Chim. France 1599 (1970) ou C. R. Acad. Sci., Ser. C, *267*, (25), 1726 (1968).

— Lorsque R est un radical triazol-1,2,4 yle-5 substitué en position -1 par thiazolidinyl-2 alcoyle ou par hydroxyiminoalcoyle : par action respectivement de cystéamine ou d'hydroxylamine sur un dialcoyloxyalcoyl-1 mercapto-5 triazole-1,2,4 qui peut être obtenu par analogie avec la méthode décrite par M. Kanaoka, J. Pharm. Soc. Japan, *75*, 1149 (1955), à partir d'une dialcoyloxyalcoyl-4 thiosemicarbazide.

— Lorsque R est un radical triazol-1,2,4 yle-5 substitué en position -1 par dihydroxy-2,3 propyle ou dihydroxy-1,3 propyle-2 (éventuellement protégés sous forme d'acétal cyclique), ou par un radical de formule générale (XVIa) ou (XVIb) : par analogie avec la méthode décrite par M. Kanaoka, J. Pharm. Soc. Japan, *75*, 1149 (1955).

— Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou un radical alcoyloxycarbonyl-3 triazol-1,2,4 yle-5 ou triazol-1,2,4 yle-5 substituée en position -1, par acyloxyalcoyle (éventuellement substitué) : par acylation respectivement de la dioxo-5,6 hydroxyalcoyl-1 mercapto-3 tétrahydro-1,4,5,6 triazine-1,2,4, de la dioxo-5,6 hydroxyalcoyl-2 mercapto-3 tétrahydro-1,2,5,6 triazine-1,2,4, de l'alcoyloxycarbonyl-3 hydroxyalcoyl-1 mercapto-5 triazole-1,2,4 ou de l'hydroxyalcoyl-1 mercapto-5 triazole-1,2,4 dont le radical mercapto a été préalablement protégé [par exemple selon C. G. Kruse et coll., Tet. Lett. 1725 (1976)], par toute méthode connue pour acyler un alcool sans toucher au reste de la molécule, puis libération du groupement mercapto en milieu acide.

— Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou alcoyloxycarbonyl-3 triazol-1,2,4 yle-5 ou triazol-1,2,4 yle-5 substitués en position -1 par aminoalcoyle ou alcoylaminoalcoyle :

par libération de la fonction amine du produit correspondant, protégé par exemple par un groupement t. butoxycarbonyle.

— Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2, alcoyloxycarbonyl-3 triazol-1,2,4 yle-5 ou triazol-1,2,4 yle-5 substitués en position -1 par sulfoaminoalcoyle :

à partir du produit correspondant substitué par exemple par un radical t. butoxycarbonylaminoalcoyle, par analogie avec la méthode décrite dans le brevet belge 847 237.

— Lorsque R est un radical alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1 par un radical formylalcoyle ou par un radical de formule générale (XVIa) dans laquelle $R^{\beta}$ représente un atome d'hydrogène : en faisant agir un oxalate d'alcoyle sur une thiosemicarbazide, par analogie avec la méthode décrite par M. Pesson et M. Antoine, C. R. Acad. Sci. *267*, (25C), 904 (1968) ou C. R. Acad. Sci. *267* (25C), 1726 (1968).

II/ Les thiovinylcéphalosporines de formule générale (XV) dans laquelle R représente un radical dioxo-5,6 tétrahydro-1,4,5,6 triazine-1,2,4 yl-3 substitué en position -1 ou -4, dioxo-5,6 tétrahydro-1,2,5,6 triazine-1,2,4 yl-3 substitué en position -2, ou bien triazol-1,3,4 yl-5, alcoyloxycarbonyl-2 triazol-1,3,4 yl-5, triazol-1,2,4 yl-5 ou alcoyloxycarbonyl-3 triazol-1,2,4 yl-5 substitués en position -1, par un radical alcoyle contenant 2 à 4 atomes de carbone substitué par un groupement carbamoyloxy ou acyloxy (dont la partie acyle est éventuellement substituée par un radical amino, alcoylamino ou dialcoylamino), et $R^a_5$, $R^b_5$ et

$R^{o}_{2}$ ont les définitions correspondantes, qui sont les dérivés fonctionnels du produit de formule générale (XV) dans laquelle R est un radical —®—alk'—OH choisi parmi dioxo-5,6 hydroxyalcoyl-1 (ou -4) tétrahydro-1,4,5,6 triazine-1,2,4 yl-3, dioxo-5,6 hydroxyalcoyl-2 tétrahydro-1,2,5,6 triazine-1,2,4 yl-3, hydroxyalcoyl-1 triazol-1,3,4 (ou -1,2,4) yl-5, alcoyloxycarbonyl-3, hydroxyalcoyl-1 triazol-1,2,4 yl-5 et $R^{a}_{5}$, $R^{b}_{5}$ et $R^{o}_{2}$ sont définis comme ci-dessus, peuvent être obtenues par carbamatation ou estérification d'un alcool de formule générale

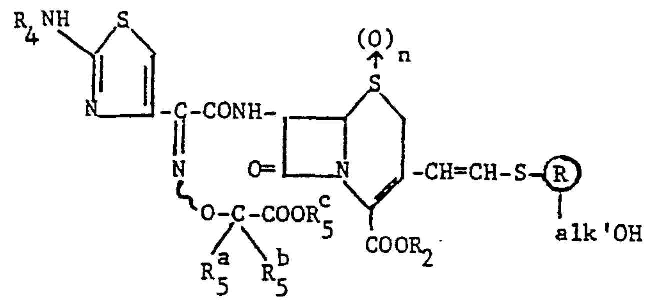

(XVa)

dans laquelle $R_2$ est défini comme $R^{o}_{2}$ ou représente un radical protecteur, $R_4$, $R^{a}_{5}$, $R^{b}_{5}$, $R^{c}_{5}$ et n sont définis comme dans la formule générale (I) et —®—alk'—OH est défini comme ci-dessus, par toute méthode connue pour obtenir un carbamate ou un ester à partir d'un alcool sans toucher au reste de la molécule, puis s'il y a lieu, réduction du sulfoxyde obtenu et élimination des radicaux protecteurs.

L'estérification s'effectue à une température comprise entre − 50 °C et la température de reflux du mélange réactionnel, notamment par condensation de l'anhydride de l'acide (ou d'un autre dérivé réactif, par exemple halogénure) dans un solvant organique inerte tel qu'un éther (par exemple tétrahydrofuranne), un solvant chloré (par exemple dichlorométhane), ou un mélange de tels solvants, en présence d'une base azotée comme la pyridine, la diméthylamino-4 pyridine ou une trialcoylamine (triéthylamine) ou d'un agent alcalin de condensation (par exemple bicarbonate de sodium) puis, le cas échéant, réduction du S-oxyde obtenu et élimination des groupements protecteurs selon les méthodes décrites précédemment.

L'obtention du carbamate s'effectue par toute méthode connue qui n'altère pas le reste de la molécule. On opère notamment par action d'isocyanate de chlorosulfonyle ou de trichloracétyle dans un solvant organique inerte, par exemple le tétrahydrofuranne ou l'acétonitrile, à une température comprise entre − 80 et 20 °C, puis élimine les groupements protecteurs.

III/ Les thiovinylcéphalosporines de formule générale (XV) dans laquelle R représente un radical dioxo-5,6 tétrahydro-1,4,5,6 triazine-1,2,4 yl-3 substitué en position -1 ou -4, dioxo-5,6 tétrahydro-1,2,5,6 triazine-1,2,4 yl-3 substitué en position -2, ou bien triazol-1,3,4 yl-5, alcoyloxycarbonyl-2 triazol-1,3,4 yl-5, triazol-1,2,4 yl-5 ou alcoyloxycarbonyl-3 triazol-1,2,4 yl-5 substitués en position -1, par un radical alcoyle contenant 2 à 4 atomes de carbone substitué par un groupement sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido ou dialcoyluréido, ou représente un radical thiadiazol-1,3,4 yl-5 substitué par un radical acylamino ou acylaminoalcoyle, ou représente un radical oxadiazol-1,3,4 yl-5 substitué par un radical acylaminoalcoyle, ou représente un radical tétrazolyl-5 substitué en position -1 par un radical alcoyle contenant 2 à 4 atomes de carbone substitué par un groupement acylamino, sulfamoylamino, sulfoamino, uréido, alcoyluréido ou dialcoyluréido et $R^{a}_{5}$, $R^{b}_{5}$ et $R^{o}_{2}$ ont les définitions correspondantes, qui sont tous des dérivés fonctionnels de l'amine qui leur correspond, peuvent être obtenues par traitement d'une amine de formule générale

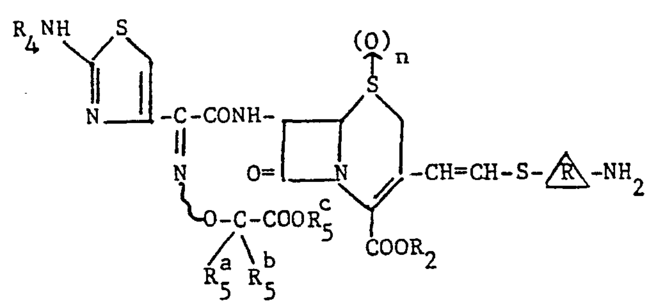

(XVb)

dans laquelle $R^a_5$, $R^b_5$, $R^c_5$, $R_4$ et n sont définis comme précédemment, pour les produits de formule générale (I), $R_2$ est défini comme $R^o_2$ ou représente un radical protecteur et —R—$NH_2$ représente un radical dioxo-5,6 tétrahydro-1,4,5,6 triazine-1,2,4 yl-3 substitué en position -1 ou -4, dioxo-5,6 tétrahydro-1,2,5,6 triazine-1,2,4 yl-3 substitué en position -2, ou bien triazol-1,3,4 yl-5, alcoyloxycarbonyl-2 triazol-1,3,4 yl-5, triazol-1,2,4 yl-5 ou alcoyloxycarbonyl-3 triazol-1,2,4 yl-5 substitués en position -1, par un radical aminoalcoyle dont la partie alcoyle contient 2 à 4 atomes de carbone, ou un radical thiadiazol-1,3,4 yl-5 substitué par un radical amino ou aminoalcoyle, ou un radical oxadiazol-1,3,4 yl-5 substitué par un radical aminoalcoyle, ou un radical tétrazolyl-5 substitué en position -1 par un radical aminoalcoyle dont la partie alcoyle contient 2 à 4 atomes de carbone, par toute méthode connue en soi pour former une fonction amide, sulfamide, carbamate ou urée sans toucher au reste de la molécule, puis le cas échéant réduction du sulfoxyde et élimination des groupements protecteurs.

Il est entendu que les produits qui contiennent un groupement sulfo, sulfonyle ou sulfamoyle sont préparés de préférence à partir d'un produit de formule générale (XVb) dans laquelle n = 0.

Par ailleurs, lorsque l'on veut préparer un produit dont le radical R contient un groupement amino ou hydroxy, il est nécessaire de protéger ces radicaux dans le réactif utilisé.

Lorsque l'on veut préparer un produit de formule générale (XV) dans laquelle le radical R contient un substituant alcoylsulfonylamino, sulfamoylamino, acylamino (substitué ou non), alcoyloxycarbonylamino ou dialcoyluréido, la réaction est effectuée avantageusement par action, respectivement, du dérivé chlorosulfonyle, du chlorure d'acide, de chloroformiate ou du chlorure de dialcoylcarbamoyle correspondant dans les conditions décrites précédemment pour la réaction du chlorure de l'acide de formule générale (XI) sur l'amino-7 céphalosporine de formule générale (X).

Lorsque l'on veut préparer un produit de formule générale (XV) dans laquelle le radical R contient un substituant sulfoamino, alcoylsulfonylamino ou acylamino (substitué ou non), on peut effectuer la réaction au moyen de l'anhydride de l'acide correspondant, dans les conditions décrites précédemment pour faire réagir le produit de formule générale (XI) sous forme d'anhydride.

Lorsque l'on veut obtenir un produit de formule générale (XV) pour lequel R contient un radical acylamino (substitué ou non), il est également possible de faire agir l'acide correspondant, dans les conditions opératoires décrites précédemment pour l'emploi de l'acide de formule générale (XI).

Lorsque l'on veut obtenir un produit de formule générale (XV) dans laquelle R contient un radical uréido ou alcoyluréido, on fait agir respectivement un isocyanate alcalin ou un isocyanate d'alcoyle sur le produit correspondant de formule générale (XVb) en milieu hydroorganique ou organique (par exemple dans le tétrahydrofuranne) à une température comprise entre − 20 et 60 °C.

La réduction et l'élimination des radicaux protecteurs s'effectuent dans les conditions décrites précédemment.

IV/ Les thiovinylcéphalosporines de formule générale (XV) dans laquelle R représente un radical dioxo-5,6 tétrahydro-1,4,5,6 triazine-1,2,4 yl-3 substitué en position -1 ou -4, dioxo-5,6 tétrahydro-1,2,5,6 triazine-1,2,4 yl-3 substitué en position -2, ou bien triazol-1,3,4 yl-5, alcoyloxycarbonyl-2 triazol-1,3,4 yl-5, triazol-1,2,4 yl-5 ou alcoyloxycarbonyl-3 triazol-1,2,4 yl-5 substitués en position -1, par un radical de formule générale (XVIc) ou par un radical hydroxyiminoalcoyle ou alcoyloxyiminoalcoyle dont la partie iminoalcoyle contient 2 à 5 atomes de carbone ou bien représente un radical tétrazolyl-5 substitué en position -1, par un radical hydroxyiminoalcoyle ou alcoyloxyiminoalcoyle dont la partie iminoalcoyle contient 2 à 5 atomes de carbone et $R^a_5$, $R^b_5$ et $R^o_2$ ont les définitions correspondantes, qui sont des dérivés d'addition du produit de formule générale (XV) dans laquelle R est l'un des hétérocycles cités ci-dessus substitué par un radical formylalcoyle (ou sa forme hydrate), peuvent être obtenues à partir d'un aldéhyde de formule générale

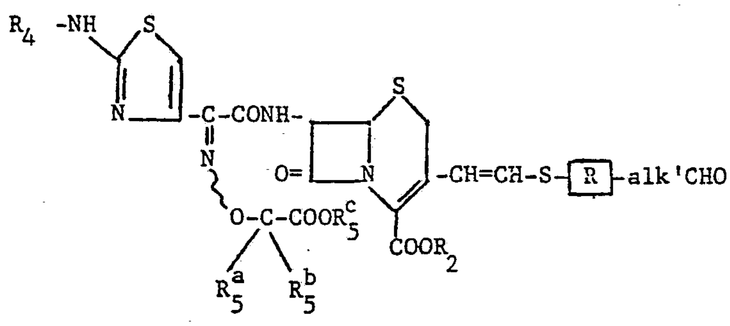

(XVc)

dans laquelle $R^a_5$, $R^b_5$ et $R^c_5$ sont définis comme précédemment pour les produits de formule générale (I), $R_2$ et $R_4$ sont définis comme dans la formule générale (I) ou représentent un atome d'hydrogène et —R—alk'CHO représente un radical dioxo-5,6 formylalcoyl-1 (ou 4) tétrahydro-1,4,5,6 triazine-1,2,4 yl-3,

formylalcoyl-1 triazol-1,3,4 (ou -1,2,4) yl-5, alcoyloxycarbonyl-2 formylalcoyl-1 triazol-1,3,4 yl-5, alcoyloxycarbonyl-3 formylalcoyl-1 triazol-1,2,4 yl-5 ou formylalcoyl-1 tétrazolyl-5, par addition respectivement de cystéamine, d'un alcool, d'hydroxylamine ou d'une alcoyloxyamine selon les méthodes connues pour former des dérivés d'addition de fonctions carbonylées, puis s'il y a lieu élimination des radicaux protecteurs.

La réaction s'effectue généralement dans un solvant organique à une température comprise entre 20 °C et la température de reflux du mélange réactionnel.

Les solvants organiques sont choisis en fonction de la solubilité des produits. Lorsque l'on met en œuvre un produit de formule générale (XVc) dans laquelle $R^c_5$, $R_4$ et $R_2$ sont autres que l'hydrogène, on utilise avantageusement des solvants tels que le tétrahydrofuranne, l'acétonitrile, les alcools, les cétones. Lorsque l'on met en œuvre un produit de formule générale (XVc) dans laquelle $R^c_5$, $R_4$ et $R_2$ sont des atomes d'hydrogène, on opère avantageusement dans des solvants tels que la pyridine, le diméthylsulfoxyde ou le diméthylformamide.

Lorsque l'on veut préparer un produit de formule générale (XV) pour lequel le radical R contient un substituant de formule générale (XVIc), on opère en milieu acide.

V/ Les thiovinyl-3 céphalosporines de formule générale (XV) dans laquelle $R^o_2$ représente un radical de formule générale (II) dans laquelle $R_9$ et $R_8$ sont définis comme précédemment, peuvent aussi être obtenues par estérification d'un produit de formule générale (XV) dans laquelle $R^o_2$ représente un atome d'hydrogène et dont la fonction amine et la fonction acide —$COOR^c_5$ ont été préalablement protégées, par toute méthode connue en soi pour préparer un ester à partir d'un acide sans toucher au reste de la molécule.

On opère notamment dans les conditions décrites précédemment pour la préparation de produits de formule générale (VIII) dans laquelle $R_2$ est un radical de formule générale (II).

Les isomères des produits de formules générales (I), (IV), (XV), (XVIII) et (XVIIIa) peuvent être séparés par cristallisation ou par chromatographie.

Les nouveaux produits selon l'invention et les produits de formule générale (XV) peuvent être éventuellement purifiés par des méthodes physiques telles que la cristallisation ou la chromatographie.

Les dérivés de la céphalosporine de formule générale (XV) tels que définis en α) et leurs sels pharmaceutiquement acceptables présentent des propriétés antibactériennes particulièrement intéressantes. Ils manifestent une activité remarquable in vitro et in vivo sur les germes Gram-positifs et Gram-négatifs.

In vitro, ils se sont montrés actifs à une concentration comprise entre 0,5 et 10 $\mu g/cm^3$ sur des souches de staphylocoques sensibles à la pénicilline G (Staphylococcus aureus Smith), et à une concentration comprise entre 0,01 et 2 $\mu g/cm^3$ sur Escherichia coli souche NIHJ. De plus certains se sont montrés actifs à une concentration comprise entre 2 et 125 $\mu g/cm^3$ sur Pseudomonas aeruginosa.

In vivo ils se sont montrés actifs sur les infections expérimentales de la souris à Staphylococcus aureus Smith (sensible à la pénicilline G) à une dose comprise entre 0,5 et 15 mg/kg par jour par voie sous-cutanée, et à Escherichia coli (souche NIHJ) des doses comprises entre 0,01 et 10 mg/kg par jour par voie sous-cutanée.

Par ailleurs leur $DL_{50}$ est comprise entre 1 g/kg et des doses supérieures à 2,5 g/kg par voie sous-cutanée chez la souris.

D'un intérêt tout particulier sont les produits de formule générale (I) dans laquelle n est égal à 0, les symboles $R^a_5$ et $R^b_5$ représentent des radicaux alcoyle, le symbole $R^c_5$ représente un radical protecteur d'acide, le symbole $R_4$ représente un radical protecteur d'amine et le symbole $R_2$ représente un radical protecteur d'acide, et plus spécialement les isomères oxoéthyl-3 bicyclooctène-2.

L'exemple suivant, donné à titre non limitatif, montre comment l'invention peut être mise en pratique.

Dans cet exemple les produits sont cités selon la nomenclature des Chemical Abstracts. Il est entendu que tous les produits selon la présente invention présentent la stéréochimie donnée par la formule générale partielle

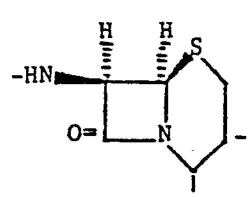

(XXIII)

Exemple

On agite pendant 1 heure à 20 °C un mélange de 6,45 g de benzhydryloxycarbonyl-2 (diméthylamino-2 vinyl)-3 {[(t. butoxycarbonyl-2 propyl-2) oxyimino]-2 (tritylamino-2 thiazolyl-4)-2 acétamido}-7 oxo-8 thia-5 aza-1 bicyclo [4.2.0.] octène-2, isomère syn, forme E (produit Ia) en solution dans 100 $cm^3$ d'acétate

d'éthyle et 64 $cm^3$ d'acide chlorhydrique 1N. On décante, lave par 100 $cm^3$ d'eau, 100 $cm^3$ d'eau saturée de bicarbonate de sodium et 100 $cm^3$ d'eau saturée de chlorure de sodium, sèche sur sulfate de sodium, filtre et concentre à sec à 20 °C sous 20 mm de mercure (2,7 kPa). On recueille 4,95 g de benzhydryloxycarbonyl-2 {[(t. butoxycarbonyl-2 propyl-2) oxyimino]-2 (tritylamino-2 thiazolyl-4)-2 acétamido}-7 formylméthyl-3 oxo-8 thia-5 aza-1 bicyclo [4.2.0] octène-2, isomère syn (produit 1b) brut sous la forme d'une meringue brune.

Spectre infra-rouge ($CHBr_3$), bandes caractéristiques ($cm^{-1}$) 3 400, 3 270, 2 720, 1 770, 1 725, 1 685, 1 525, 1 495, 1 450, 1 370, 755, 700.

Spectre de RMN du proton (350 MHz, $CDCl_3$, δ en ppm, J en Hz) 1,45 (s, 9H, $-C(CH_3)_3$) ; 1,64 et 1,67 (2s, 6H, $(-CH_3)_2$) ; 3,25 et 3,54 (2d, J = 18, 2H, $-SCH_2-$) ; 3,50 et 3,73 (2d, J = 16, 2H, $-C\underline{H}_2CHO$) ; 5,10 (d, J = 4, 1H, H en 6) ; 6,06 (dd, J = 4 et 9, 1H, H, en 7) ; 6,77 (s, 1H, H du thiazole) ; 6,90 (s, 1H, $-COOCH<$) ; 8,22 (d, J = 9, 1H, $-CONH-$) ; 9,58 (s, 1H, $-CHO$).

A une solution à 80 °C sous azote de 6 g de benzhydryl-oxycarbonyl-2 [[[(t. butoxycarbonyl-2 propyl-2) oxyimino]-2 (tritylamino-2 thiazolyl-4)-2 acétamido]]-7 méthyl-3 oxo-8 thia-5 aza-1 bicyclo [4.2.0] octène-2, isomère syn, dans 64 $cm^3$ de diméthylformamide, on ajoute sous agitation 1,9 $cm^3$ de t. butoxy bis-diméthylaminométhane et poursuit la réaction pendant 15 minutes. On verse ensuite le mélange dans un mélange de 200 $cm^3$ d'acétate d'éthyle et 200 $cm^3$ d'eau, décante, lave par 3 fois 100 $cm^3$ d'eau et 100 $cm^3$ d'eau saturée de chlorure de sodium, sèche sur sulfate de sodium, filtre et concentre à sec à 20 °C sous 20 mm de mercure (2,7 kPa). On obtient ainsi 6,5 g de produit (la) brut sous la forme d'une meringue brune.

On purifie un échantillon (2,2 g) par chromatographie sur une colonne (diamètre 4 cm, hauteur 20 cm) de gel de silice Merck (0,04-0,06) en éluant par un mélange cyclohexane-acétate d'éthyle 65/35 (vol.) sous une pression de 40 kPa et en recueillant des fractions de 50 $cm^3$. Les fractions 14 à 24 sont concentrées à sec. On obtient 1,2 g de benzhydryloxycarbonyl-2 (diméthylamino-2 vinyl)-3, [[[t. butoxycarbonyl-2 propyl-2) oxyimino]-2 (tritylamino-2 thiazolyl-4)-2 acétamido]]-7 oxo-8 thia-5 aza-1 bicyclo [4.2.0.] octène-2 isomère syn, forme E.

Spectre infra-rouge ($CHBr_3$), bandes caractéristiques ($cm^{-1}$) 3 400, 3 280, 1 770, 1 720, 1 680, 1 610, 1 525, 1 490, 1 450, 1 370, 940, 750, 735.

Spectre de RMN du proton (350 MHz, $CDCl_3$, δ en ppm, J en Hz) 1,45 (s, 9H, $-C(CH_3)_3$) ; 1,64 et 1,71 (2s, 6H, $=N-O-C(CH_3)_2-$) ; 2,93 (s, 6H, $-N(CH_3)_2$) ; 3,20 et 3,30 (2d, J = 14, 2H, $-S-CH_2-$) ; 5,18 (d, J = 4, 1H, —H en 6) ; 5,71 (dd, J = 4 et 9, 1H, —H en 7) ; 6,61 et 6,82 (2d, J = 14, 2H, $-CH=CH-S-$) ; 6,88 (s, 1H, H thiazole ; 6,92 (s, 1H, $-COO-C\underline{H}(C_6H_5)_2$) ; 6,92 (s large, 1H, $-N\underline{H}-C(C_6H_5)_3$) ; 8,28 (d, J = 9, 1H, $-CO-NH-$).

Le benzhydryloxycarbonyl-2 [[[(t. butoxycarbonyl-2 propyl-2) oxyimino]-2 (tritylamino-2 thiazolyl-4)-2 acétamido]]-7 méthyl-3 oxo-8 thia-5 aza-1 bicyclo [4.2.0.] octène-2, isomère syn peut être préparé de la manière suivante :

A une solution de 25,58 g d'acide (t. butoxycarbonyl-2 propyl-2 oxyimino)-2 (tritylamino-2 thiazolyl-4)-2 acétique préparé selon le brevet belge 876 541 dans un mélange de 250 $cm^3$ de dichlorométhane et 13,9 $cm^3$ de diméthylacétamide, on ajoute à −10 °C, en 30 minutes et sous agitation 40 $cm^3$ d'une solution 1,3 M de phosgène dans du chlorobenzène. On agite encore pendant 3 heures à −10 °C et ajoute goutte à goutte en 1 heure 15 une solution de 16,29 g d'amino-7 benzhydryloxycarbonyl-2 méthyl-3 oxo-8 thia-5 aza-1 bicyclo [4.2.0] octène-2 dans 250 $cm^3$ de dichlorométhane. Après 1 heure 15 à − 10 °C, on lave le mélange par 200 $cm^3$ d'une solution aqueuse à 2 % de bicarbonate de sodium et 200 $cm^3$ d'eau, sèche sur sulfate de sodium et concentre à sec à 30 °C sous 20 mm de mercure (2,7 kPa). Le résidu est chromatographié sur une colonne de 200 g de gel de silice Merck (0,05-0,2) (diamètre de la colonne : 4 cm). On élue par un mélange cyclohexane — acétate d'éthyle 70-30 (en volumes) en recueillant des fractions de 120 $cm^3$. Les fractions 6 à 10 sont concentrées à sec à 30 °C sous 20 mm de mercure (2,7 kPa). On recueille 8,5 g de benzhydryloxycarbonyl-2 [(t. butoxycarbonyl-2 propyl-2 oxyimino)-2 (tritylamino-2 thiazolyl-4)-2 acétamido]-7 méthyl-3 oxo-8 thia-5 aza-1 bicyclo [4.2.0] octène-2, isomère syn, sous la forme d'une meringue jaune.

Spectre infra-rouge ($CHBr_3$), bandes caractéristiques ($cm^{-1}$) 3 400, 3 270, 1 790, 1 725, 1 685, 1 525, 1 500, 1 450, 1 380, 1 370, 760, 740.

Les produits selon l'invention peuvent être utilisés de la manière suivante :

## Exemple de référence

A une solution refroidie à 5 °C de 4,45 g de produit (1b) dans 50 $cm^3$ de pyridine, on ajoute 1,31 g de chlorure de p. toluènesulfonyle. On laisse remonter la température à 20 °C en 30 minutes, agite pendant 1 heure à 20 °C, verse dans 300 $cm^3$ d'eau glacée, décante l'eau et reprend le produit pâteux insoluble dans 300 $cm^3$ d'acétate d'éthyle. On lave par 100 $cm^3$ d'acide chlorhydrique 1N, 100 $cm^3$ d'une solution saturée de bicarbonate de sodium et 100 $cm^3$ d'une solution saturée de chlorure de sodium, sèche sur sulfate de sodium, filtre et concentre à sec à 20 °C sous 20 mm de mercure (2,7 kPa). Le produit ainsi obtenu est utilisé tel quel pour la suite de la synthèse. 1 g de ce produit est purifié par chromatographie sur une colonne de 20 g de gel de silice (0,05-0,2) (diamètre de la colonne : 1,7 cm) en éluant par un mélange de cyclohexane et d'acétate d'éthyle 80-20 (en volumes) et en recueillant des fractions de

50 $cm^3$. Les fractions 4 à 12 contenant le produit pur sont évaporées à sec sous pression réduite (30 mm de mercure ; 4 kPa) à 30 °C. On obtient 0,43 g de benzhydryloxycarbonyl-2 {[(t. butoxycarbonyl-2 propyl-2) oxyimino]-2 (tritylamino-2 thiazolyl-4)-2 acétamido}-7 oxo-8 (tosyloxy-2 vinyl)-3 thia-5 aza-1 bicyclo [4.2.0] octène-2, isomère syn, produit (1d) mélange des formes E et Z (dans des proportions — déterminées par RMN — de 75 % de forme E et 25 % de forme Z) sous la forme d'un solide jaune pâle.

Spectre infra-rouge ($CHBr_3$), bandes caractéristiques ($cm^{-1}$) 3 400, 3 260, 1 790, 1 720, 1 680, 1 630, 1 595, 1 580, 1 520, 1 490, 1 450, 1 380, 1 370, 1 190, 1 180, 1 070, 835, 750.

Spectre de RMN du proton (350 MHz, $CDCl_3$, δ en ppm, J en Hz) mélange d'isomères E et Z dans les proportions 75/25

forme E : 1,45 (s, 9H, $—C(CH_3)_3$) ; 1,62 et 1,67 (2s, 6H, $=N—O—C(CH_3)_2$) ; 2,48 (s, 3H, $—CH_3$ tosyl) ; 3,42 et 3,50 (2d, J = 18, 2H, $—S—CH_2—$) ; 5,08 (d, J = 4, 1H, —H en 6) ; 6,00 (dd, J = 4 et 9, 1H, —H en 7) ; 6,78 (s, 1H, H thiazole) ; 6,88 (mf, 1H, $—N\underline{H}—C(C_6H_5)_3$) ; 6,90 et 6,97 (2d, J = 12, 2H, —CH=CH—S—) ; 6,92 (s, 1H, $—COO—C\underline{H}(C_6H_5)_2$ ; 8,20 (d, J = 9, 1H, —CO—NH—)

forme Z : 6,20 et 6,47 (2d, J = 7, —CH=CH—S— Z) ; 2,45 (s, $—CH_3$ tosyl).

A une solution refroidie à 0 °C de 3 g de produit (1d) dans 50 $cm^3$ de dichlorométhane, on ajoute goutte à goutte en 25 minutes une solution de 0,464 g d'acide m. chloroperbenzoïque à 90 % dans 10 $cm^3$ de dichlorométhane. On agite pendant 1 heure à 0 °C, dilue par 500 $cm^3$ d'acétate d'éthyle, lave par 2 fois 100 $cm^3$ d'une solution à 2 % de bicarbonate de sodium, 2 fois 100 $cm^3$ d'eau et 100 $cm^3$ d'eau saturée de chlorure de sodium, sèche sur sulfate de sodium, filtre et concentre à sec à 20 °C sous 20 mm de mercure (2,7 kPa). Le résidu est chromatographié sur une colonne de 60 g de gel de silice Merck (0,06-0,2) (diamètre de la colonne : 2 cm, hauteur : 20 cm), on élue par 1 litre d'un mélange cyclohexane-acétate d'éthyle (70-30 en volumes) en recueillant des fractions de 60 $cm^3$. Les fractions 5 à 14 sont concentrées à sec à 20 °C sous 20 mm de mercure (2,7 kPa), on recueille 1,9 g de benzhydryloxycarbonyl-2 {[(t. butoxycarbonyl-2 propyl-2) oxyimino]-2 (tritylamino-2 thiazolyl-4)-2 acétamido}-7 oxo-8 oxyde-5 (tosyloxy-2 vinyl)-3 thia-5 aza-1 bicyclo [4.2.0.] octène-2, isomère syn, forme E (produit 1e) sous la forme d'une meringue jaune dont les caractéristiques sont les suivantes :

Spectre infra-rouge ($CHBr_3$), bandes caractéristiques ($cm^{-1}$) 3 380, 1 800, 1 720, 1 680, 1 590, 1 580, 1 510, 1 490, 1 445, 1 375, 1 190, 1 175, 1 070, 730

Spectre de RMN du proton (350 MHz, DMSO $d_6$, δ en ppm, J en Hz) 1,37 (s, 9H, $—C(CH_3)_3$) ; 1,45 et 1,46 (2s, 6H, $—OC(CH_3)_2—$) ; 2,44 (s, 3H, $—CH_3$ tosyle) ; 3,60 et 4,41 (2d, J = 18, 2H, $—SCH_2—$) ; 5,06 (d, J = 4, 1H, H en 6) ; 5,96 (dd, J = 4 et 9, 1H, H en 7) ; 6,75 (d, J = 13, 1H, $—C\underline{H}=CHS—$) ; 6,73 (s, 1H, H du thiazole) ; 6,93 (s, 1H, —COOCH—) ; 7,48 et 7,84 (ab, 2H, J = 9) ; 8,16 (d, J = 9, 1H, —CONH—) ; 8,73 (s, 1H, $—N\underline{H}\ C(C_6H_5)_3$).

On chauffe à 60 °C sous azote pendant 4 heures, sous agitation, un mélange de 6,79 g de produit (1e), 60 $cm^3$ de diméthylformamide, 1,68 g de (diméthoxy-2,2 éthyl)-4 dioxo-5,6 thioxo-3 perhydrotriazine-1,2,4 et 1,25 $cm^3$ de N,N-diisopropyléthylamine. On dilue le mélange par 250 $cm^3$ d'acétate d'éthyle, lave par 3 fois 125 $cm^3$ d'eau et 2 fois 125 $cm^3$ d'une solution saturée de chlorure de sodium, sèche sur sulfate de sodium, filtre et concentre à sec à 20 °C sous 20 mm de mercure (2,7 kPa). Le résidu obtenu après ce traitement est chromatographié sur une colonne de 125 g de gel de silice Merck (0,06-0,2) (diamètre de la colonne : 3 cm, hauteur : 43 cm). On élue par 1,5 litre d'un mélange cyclohexane-acétate d'éthyle 50-50 (en volumes) et 1 litre d'acétate d'éthyle en recueillant des fractions de 100 $cm^3$. Les fractions 16 à 21 sont concentrées à sec à 25 °C sous 20 mm de mercure (2,7 kPa), on obtient 5,5 g de benzhydryloxycarbonyl-2 {[(t. butoxycarbonyl-2 propyl-2) oxyimino]-2 (tritylamino-2 thiazolyl-4)-2 acétamido}-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo [4.2.0] octène-2, isomère syn, forme E (produit 1f) sous la forme d'une meringue brune.

Spectre infra-rouge ($CHBr_3$), bandes caractéristiques ($cm^{-1}$) 1 800, 1 720, 1 690, 1 585, 1 510, 1 495, 1 445, 1 370, 1 080, 1 060, 1 040, 940, 750, 700

Spectre de RMN du proton (350 MHz, DMSO $d_6$, δ en ppm, J en Hz 1,35 (s, 9H, $—C(CH_3)_3$) ; 1,44 et 1,45 (2s, 6H, $=N—O—C(CH_3)_2—$) ; 3,32 (s, 6H, $(—OCH_3)_2$) ; 3,65 et 4,36 (2d, J = 18, 2H, $—S—CH_2—$) ; 3,95 (d, J = 5, 2H, $>N—CH_2—$) ; 4,56 (t, J = 5, 1H, $—CH<^{O—}_{O—}$) ; 5,09 (d, J = 5, 1H, —H en 6,) ; 5,95 (dd, J = 5 et 9, 1H, —H en 7) ; 6,78 (s, 1H, —H thiazole) ; 7 (s, 1H, $—COO—C\underline{H}(C_6H_5)_2$) ; 7,02 et 7,1 (2d, J = 16, 2H, —CH=CH—S—) ; 8,23 (d, J = 9, 1H, —CO—NH—) ; 8,73 (s, 1H, $—N\underline{H}—C(C_6H_5)_3$) ; 12,65 (s, 1H, =N—NH—CO— ou =N—N=C—OH).
|

On traite à − 5 °C pendant 1 heure 1/2 sous agitation une solution de 5,37 g de produit (1f) dans 45 $cm^3$ de dichlorométhane et 1,79 $cm^3$ de N,N-diméthylacétamide par 0,79 $cm^3$ de trichlorure de phosphore. Le résidu obtenu après traitement est chromatographié sur une colonne de 80 g de gel de silice Merck (0,06-0,2) (diamètre de la colonne : 2 cm, hauteur : 20 cm), on élue par 250 $cm^3$ d'un mélange cyclohexane-acétate d'éthyle 40-60 (en volumes) et 1,5 litre d'un mélange 30-70 en recueillant des fractions de 100 $cm^3$. Les fractions 5 à 14 sont concentrées à sec à 25 °C sous 20 mm de mercure, on obtient 4,02 g de benzhydryloxycarbonyl-2 {[(t. butoxycarbonyl-2 propyl-2) oxyimino]-2 (tritylamino-2 thiazolyl-4)-2 acétamido}-7 {[(diméthoxy-2,2 éthyl)-4 dioxo-5,6 tétrahydro-1,4,5,6 triazine-1,2,4 yl-3] thio-2 vinyl}-3 oxo-8 thia-5 aza-1 bicyclo [4.2.0] octène-2, isomère syn, forme E (produit 1g) sous la forme d'une meringue brun clair.

Spectre infra-rouge (KBr), bandes caractéristiques ($cm^{-1}$) 1 790, 1 720, 1 690, 1 585, 1 520, 1 495, 1 450, 1 370, 1 080, 940, 750, 700

Spectre de RMN du proton (350 MHz, DMSO $d_6$, δ en ppm, J en Hz) 1,37 (s, 9H —$C(CH_3)_3$) ; 1,42 (s, 6H, =N—O—$C(C\underline{H}_3)_2$—( ; 3,31 (s, 6H, (—$OCH_3)_2$) ; 3,64 et 3,89 (2d, J = 18, 2H, —S—$CH_2$—) ; 3,95 (d, J = 5, 2H, >N—$CH_2$—) ; 4,56 (t, J = 5, 1H, —CH<$^{O—}_{O—}$) ; 5,26 (d, J = 4, 1H, —H en 6) ; 5,77 (dd, J = 4 et 9, 1H, —H en 7) ; 6,71 (s, 1H, —H thiazole) ; 6,90 et 7,03 (2d, J = 16, 2H, —CH=CH—S—) ; 6,97 (s, 1H, —COO—$C\underline{H}(C_6H_5)_2$) ; 8,80 (s, 1H, —N$\underline{H}$—$C(C_6H_5)_3$) ; 9,39 (d, J = 9, 1H, —CO—NH—) ; 12,66 (s, 1H, =N—NH—CO— ou =N—N=C—OH).

On agite à 20 °C pendant 20 minutes une solution de 3,89 g de produit (1g) dans 39 $cm^3$ d'acide trifluoracétique. On concentre à sec à 20 °C sous 0,05 de mercure (0,007 kPa), reprend le résidu dans 100 $cm^3$ d'éther diéthylique, agite pendant 10 minutes et filtre. Le solide obtenu est traité à 50 °C pendant 45 minutes par 80 $cm^3$ d'acide formique, on ajoute 16 $cm^3$ d'eau, maintient 30 minutes à 50 °C et concentre à sec à 20 °C sous 0,05 mm de mercure (0,007 kPa). On reprend le résidu par 3 fois 150 $cm^3$ d'acétone en évaporant à chaque fois à 20 °C sous 30 mm de mercure (4 kPa) et chauffe le résidu à reflux sous agitation dans 100 $cm^3$ d'acétone. On filtre et recueille 2,15 g d'{(amino-2 thiazolyl-4)-2 [(carboxy-2 propyl-2) oxyimino]-2 acétamido}-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo [4.2.0] octène-2, isomère syn, forme E (produit 1) sous la forme d'une poudre jaune.

Spectre infra-rouge (KBr), bandes caractéristiques ($cm^{-1}$) 3 400, 3 300, 3 200, 2 200, 1 780, 1 720, 1 685, 1 585, 1 540, 1 000

Spectre de RMN du proton (350 MHz, $CF_3COOD$, δ en ppm, J en Hz) 1,85 et 1,86 (2s, 6H, —$CH_3$) ; 3,90 (s large, 2H, —$SCH_2$—) ; 5,20 (s, 2H, —$C\underline{H}_2CHO$) ; 5,40 (d, J = 4, 1H, H en 6) ; 6,12 (d, J = 4, 1H, H en 7) ; 7,23 et 7,76 (2d, J = 16, 2H, —CH=CH—) ; 7,50 (s, 1H, H du thiazole) ; 9,73 (s, 1H, —CHO).

**Revendications** (pour les Etats contractants : BE, CH, DE, FR, GB, IT, LI, LU, NL, SE)

1. Une céphalosporine caractérisée en ce qu'elle répond à la formule générale

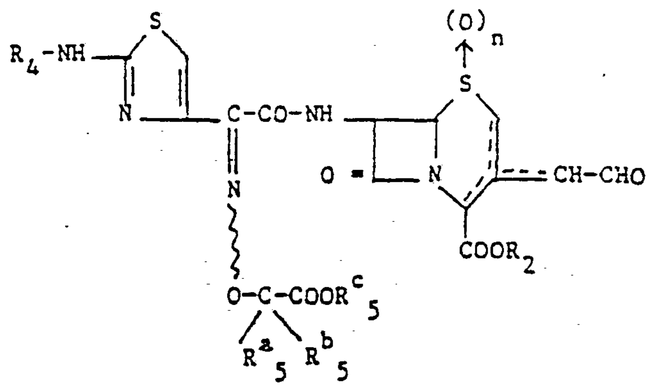

sous forme syn ou anti, dans laquelle n est égal à 0 ou 1, les radicaux $R^a_5$ et $R^b_5$, qui sont identiques ou différents, représentent des atomes d'hydrogène ou des radicaux alcoyle ou forment ensemble un radical alcoylène contenant 2 ou 3 atomes de carbone, $R^c_5$ représente un atome d'hydrogène ou un radical protecteur d'acide, $R_4$ représente un radical protecteur d'amine et le symbole $R_2$ représente un radical facilement éliminable par voie enzymatique de formule générale

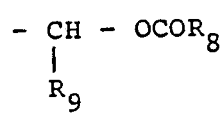

[dans laquelle $R_8$ représente un radical alcoyle ou le radical cyclohexyle et $R_9$ représente un atome d'hydrogène ou un radical alcoyle] ou un radical protecteur d'acide, les portions ou radicaux alcoyles cités ci-dessus étant droits ou ramifiés et contenant 1 à 4 atomes de carbone, le produit se présentant sous forme oxoéthyl-3 bicyclooctène-2 ou -3 ou oxoéthylidène-3 bicyclooctane lorsque n = 0 et sous forme oxoéthyl-3 bicyclooctène-2 ou oxoéthylidène-3 bicyclooctane lorsque n = 1, ainsi que les mélanges de leurs isomères.

2. Un produit selon la revendication 1 caractérisé en ce que n, $R_2$, $R^a_5$, $R^b_5$, $R^c_5$ étant définis comme dans la revendication 1, $R_4$ représente un radical t. butoxycarbonyle, trichloro-2,2,2 éthoxycarbonyle, chloracéthyle, trichloracéthyle, trityle, benzyle, dibenzyle, benzyloxycarbonyle, p. nitrobenzyloxycarbonyle, p. méthoxybenzyloxycarbonyle, formyle ou trifluoracétyle.

3. Un produit selon la revendication 1 ou la revendication 2, caractérisé en ce que les radicaux protecteurs d'acide représentés par $R^c_5$ et/ou $R_2$ sont choisis parmi les radicaux méthoxyméthyle, t. butyle, benzhydryle, benzyle, nitrobenzyle ou p. méthoxybenzyle.

4. Un dérivé de céphalosporine selon la revendication 1, 2 ou 3 caractérisé en ce que n est égal à 0, les symboles $R^a_5$ et $R^b_5$ représentent des radicaux alcoyle, les symboles $R_2$ et $R^c_5$ représentent des radicaux protecteurs d'acide et le symbole $R_4$ représente un radical protecteur d'amine.

5. Un dérivé de céphalosporine selon la revendication 4 caractérisé en ce qu'il se présente sous forme oxoéthyl-3 bicyclooctène-2.

6. Un procédé de préparation d'un produit selon la revendication 1 ou selon les revendications 2 à 5 pour lequel n est égal à 0 et les autres symboles sont définis selon l'une des revendications 1 à 5, caractérisé en ce que l'on hydrolyse une énamine de formule générale

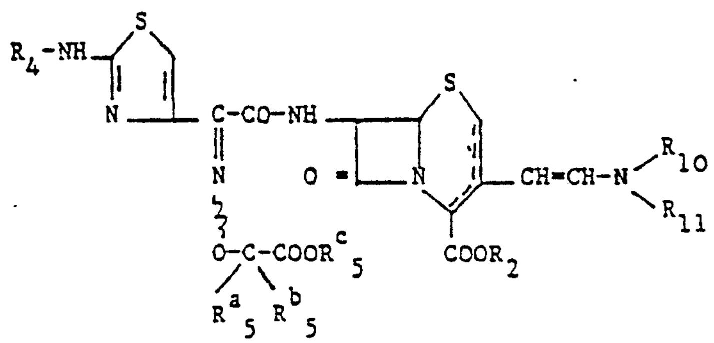

(ou un mélange de ses isomères), dans laquelle $R_2$, $R_4$, $R^a_5$, $R^b_5$ et $R^c_5$ sont définis selon la revendication 1, 2 ou 3 ($R^c_5$ étant autre que l'hydrogène) et $R_{10}$ et $R_{11}$, qui sont identiques ou différents, représentent des radicaux alcoyle (éventuellement substitués par un radical hydroxy, alcoyloxy, amino alcoylamino ou dialcoylamino) ou phényle, ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, qui se présente sous forme bicyclooctène-2 ou -3 et dont le substituant sur l'atome de carbone en position -3 du bicyclooctène présente la stéréoisomérie E ou Z, puis on élimine éventuellement le radical protecteur $R^c_5$ et/ou sépare éventuellement les isomères du produit obtenu.

7. Un procédé de préparation d'un produit selon la revendication 1 ou selon l'une des revendications 2 à 5 pour lequel n est égal à 1 et les autres symboles sont définis selon l'une des revendications 1 à 5, caractérisé en ce que l'on oxyde un produit selon la revendication 1 pour lequel n est égal à 0, puis sépare éventuellement les isomères du produit obtenu.

**Revendication** (pour l'Etat contractant AT)

Procédé de préparation d'une céphalosporine de formule générale

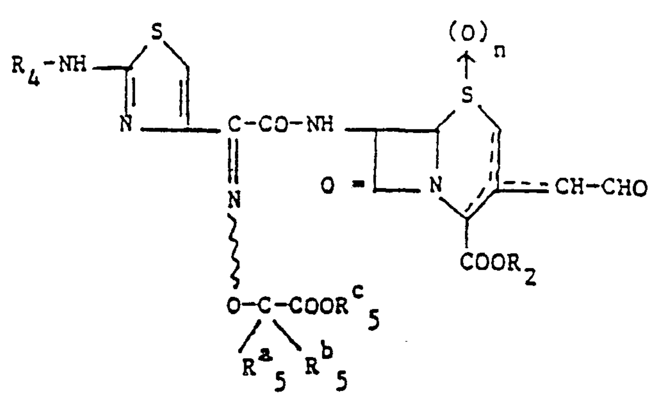

sous forme syn ou anti, dans laquelle n est égal à 0 ou 1, les radicaux $R^a_5$ et $R^b_5$, qui sont identiques ou différents, représentent des atomes d'hydrogène ou des radicaux alcoyle ou forment ensemble un radical alcoylène contenant 2 ou 3 atomes de carbone, $R^c_5$ représente un atome d'hydrogène ou un radical protecteur d'acide, $R_4$ représente un radical protecteur d'amine et le symbole $R_2$ représente un radical facilement éliminable par voie enzymatique de formule générale

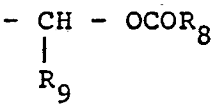

[dans laquelle $R_8$ représente un radical alcoyle ou le radical cyclohexyle et $R_9$ représente un atome d'hydrogène ou un radical alcoyle] ou un radical protecteur d'acide, les portions ou radicaux alcoyles cités ci-dessus étant droits ou ramifiés et contenant 1 à 4 atomes de carbone, le produit se présentant sous forme oxoéthyl-3 bicyclooctène-2 ou -3 ou oxoéthylidène-3 bicyclooctane lorsque n = 0 sous forme oxoéthyl-3 bicyclooctène-2 ou oxoéthylidène-3 bicyclooctane lorsque n = 1, ainsi que les mélanges de leurs isomères, caractérisé en ce que pour la préparation d'un produit dans la formule duquel n est égal à 0, on hydrolyse une énamine de formule générale

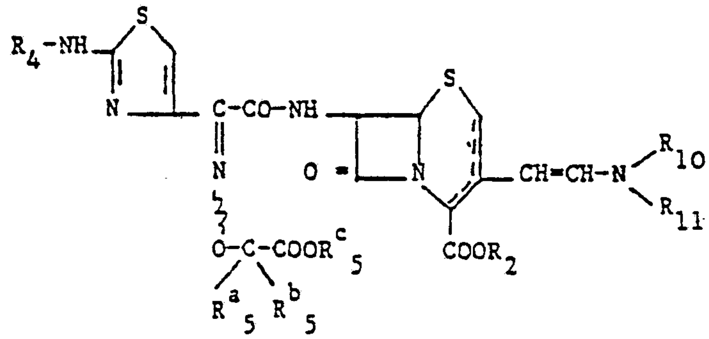

(ou un mélange de ses isomères), dans laquelle $R_2$, $R_4$, $R^a{}_5$, $R^b{}_5$ et $R^c{}_5$ sont définis comme ci-dessus ($R^c{}_5$ étant autre que l'hydrogène) et $R_{10}$ et $R_{11}$, qui sont identiques ou différents, représentent des radicaux alcoyle (éventuellement substitués par un radical hydroxy, alcoyloxy, amino, alcoylamino ou dialcoylamino) ou phényle, ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, qui se présente sous forme bicyclooctène-2 ou -3 et dont le substituant sur l'atome de carbone en position -3 du bicyclooctène présente la stéréoisomérie E ou Z, puis on élimine éventuellement le radical $R^c{}_5$ et/ou sépare éventuellement les isomères du produit obtenu, puis, pour la préparation d'un produit dans la formule duquel n est égal à 1, on oxyde le produit correspondant obtenu précédemment et pour lequel n est égal à 0, puis sépare éventuellement les isomères du produit obtenu.

**Claims** (for the Contracting States BE, CH, DE, FR, GB, IT, LI, LU, NL, SE)

1. A cephalosporin, characterised in that is corresponds to the general formula

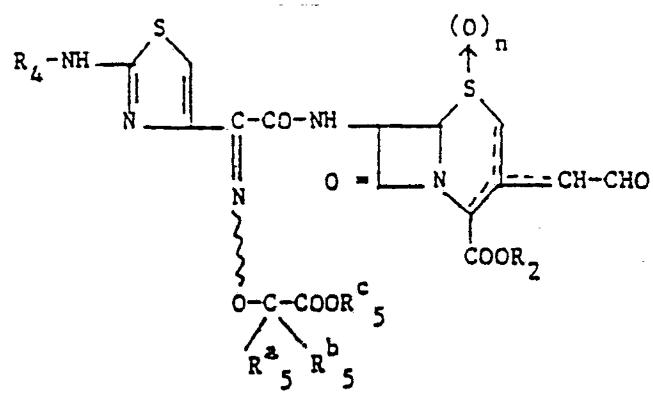

in the syn or anti form, in which n is 0 or 1, the radicals $R^a{}_5$ and $R^b{}_5$, which are identical or different, represent hydrogen atoms or alkyl radicals or together form an alkylene radical containing 2 or 3 carbon atoms, $R^c{}_5$ represents a hydrogen atom or an acid-protecting radical, $R^4$ represents an amine-protecting radical and the symbol $R_2$ represents an enzymatically easily removable radical of the general formula

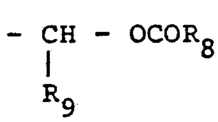

[in, which $R_8$ represents an alkyl radical or the cyclohexyl radical and $R_9$ represents a hydrogen atom or

an alkyl radical] or an acid-protecting radical, the alkyl portions or radicals mentioned above being linear or branched and containing 1 to 4 carbon atoms, and the product being in the 3-oxoethyl-bicyclooct-2-ene or 3-oxoethyl-bicyclooct-3-ene or 3-oxoethylidene-bicyclooctane form if n = 0 and in the 3-oxoethyl-bicyclooct-2-ene or 3-oxoethylidene-bicyclooctane form if n = 1, as well as mixtures of their isomers.

2. A product according to Claim 1, characterised in that n, $R_2$, $R^a_5$, $R^b_5$ and $R^c_5$ are defined as in Claim 1 and $R_4$ represents a t.-butoxycarbonyl, 2,2,2,-trichloroethoxycarbonyl, chloroacetyl, trichloroacetyl, trityl, benzyl dibenzyl, benzyloxycarbonyl, p-nitrobenzyloxycarbonyl, p-methoxybenzyloxycarbonyl, formyl or trifluoroacetyl radical.

3. A product according to Claim 1 or Claim 2, characterised in that the acid-protecting radicals represented by $R^c_5$ and/or $R_2$ are chosen from amongst the methoxymethyl, t.-butyl, benzhydryl, benzyl, nitrobenzyl or p-methoxybenzyl radicals.

4. A cephalosporin derivative according to Claim 1, 2 or 3, characterised in that n is 0, the symbols $R^a_5$ and $R^b_5$ represent alkyl radicals, the symbols $R_2$ and $R^c_5$ represent acid-protecting radicals and the symbol $R_4$ represents an amine-protecting radical.

5. A cephalosporin derivative according to Claim 4, characterised in that it is in the 3-oxoethyl-bicyclooct-2-ene form.

6. A process for the preparation of a product according to Claim 1 or according to Claims 2 to 5, for which n is 0 and the other symbols are defined according to one of Claims 1 to 5, characterised in that an enamine of the general formula

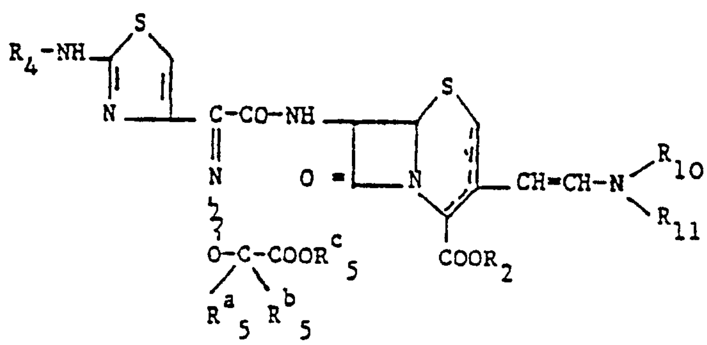

(or a mixture of its isomers), in which $R_2$, $R_4$, $R^a_5$, $R^b_5$ and $R^c_5$ are defined as in Claim 1, 2 or 3 ($R^c_5$ being other than hydrogen) and $R_{10}$ and $R_{11}$, which are identical or different, represent alkyl radicals (optionally substitued by a hydroxyl, alkoxy, amino, alkylamino or dialkylamino radical) or phenyl, or together with the nitrogen atom to which they are attached form a saturated heterocyclic ring with 5 or 6 chain members optionally containing another heteroatom chosen from amongst nitrogen, oxygen or sulphur and optionally substituted by an alkyl radical, which enamine is in the bicyclooct-2-ene or bicyclooct-3-ene form and whose substituent on the carbon atom in the 3-position of the bicyclooctene has the E or Z stereoisomeric configuration, is hydrolysed and that thereafter, where appropriate, the $R^c_5$ protective radical is removed and/or, where appropriate, the isomers of the product obtained are separated.

7. A process for the preparation of a product according to Claim 1 or according to one of Claims 2 to 5, for which n is 1 and the other symbols are defined according to one of Claims 1 to 5, characterised in that a product according to Claim 1 for which n is 0 is oxidised and thereafter, if appropriate, the isomers of the product obtained are separated.

**Claim** (for the Contracting State AT)

Process for the preparation of a cephalosporin of the general formula

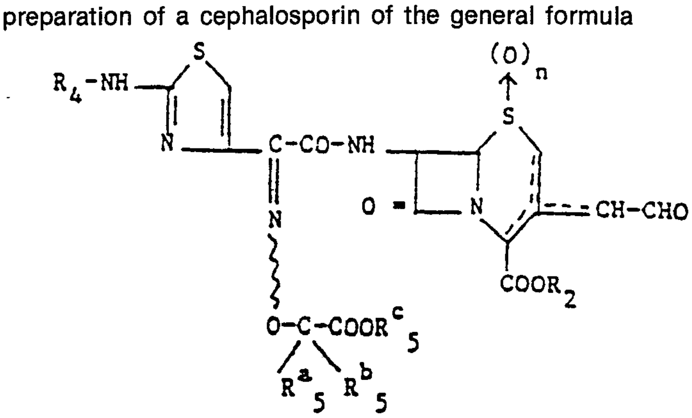

in the syn or anti form, in which n is 0 or 1, the radicals $R^a_5$ and $R^b_5$, which are identical or different, represent hydrogen atoms or alkyl radicals or together form an alkylene radical containing 2 or 3 carbon atoms, $R^c_5$ represents a hydrogen atom or an acid-protecting radical, $R_4$ represents an amine-protecting radical and the symbol $R_2$ represents an enzymatically easily removable radical of the general formula

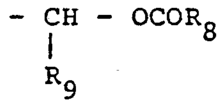

[in which $R_8$ represents an alkyl radical or the cyclohexyl radical and $R_9$ represents a hydrogen atom or an alkyl radical] or an acid-protecting radical, the alkyl portions or radicals mentioned above being linear or branched and containing 1 to 4 carbon atoms, and the product being in the 3-oxoethyl-bicyclooct-2-ene or 3-oxoethyl-bicyclooct-3-ene or 3-oxoethylidene-bicyclooctane form if n = 0 and in the 3-oxoethyl-bicyclooct-2-ene or 3-oxoethylidene-bicyclooctane form if n = 1, as well as of mixtures of their isomers, characterised in that for the preparation of a product in the formula of which n is 0, an enamine of the general formula

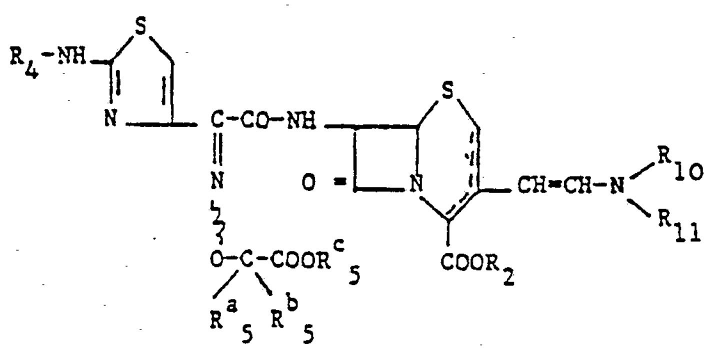

(or a mixture of its isomers), in which $R_2$, $R_4$, $R^a_5$, $R^b_5$ and $R^c_5$ are defined as above ($R^c_5$ being other than hydrogen) and $R_{10}$ and $R_{11}$, which are identical or different, represent alkyl radicals (optionally substituted by a hydroxyl, alkoxy, amino, alkylamino or dialkylamino radical) or phenyl, or together with the nitrogen atom to which they are attached form a saturated heterocyclic ring with 5 or 6 chain members optionally containing another heteroatom chosen from amongst nitrogen, oxygen or sulphur and optionally substituted by an alkyl radical, which enamine is in the bicyclooct-2-ene or bicyclooct-3-ene form and whose substituent on the carbon atom in the 3-position of the bicyclooctene has the E or Z stereoisomeric configuration, is hydrolysed and that thereafter, where appropriate, the $R^c_5$ radical is removed and/or, where appropriate, the isomers of the product obtained are separated, after which, for the preparation of a product in the formula of which n is 1, the corresponding product obtained beforehand, in which n is 0, is oxidised, and, where appropriate, the isomers of the product obtained are then separated.

**Ansprüche** (für die Vertragsstaaten : BE, CH, DE, FR, GB, IT, LI, LU, NL, SE)

1. Ein Cephalosporin, dadurch gekennzeichnet, daß es der allgemeinen Formel

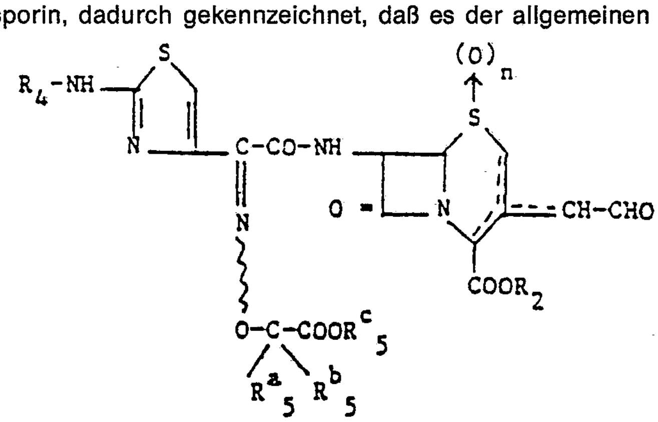

entspricht in der syn- oder anti-Form, worin n gleich 0 oder 1 ist, die Reste $R^a_5$ und $R^b_5$, welche identisch oder verschieden sind, Wasserstoffatome oder Alkylreste bedeuten oder zusammen einen Alkylenrest mit 2 oder 3 Kohlenstoffatomen bilden, $R^c_5$ ein Wasserstoffatom oder einen Säureschutzrest bedeutet, $R_4$

einen Aminschutzrest bedeutet und das Symbol $R_2$ einen auf enzymatischen Wege leicht entfernbaren rest der allgemeinen Formel

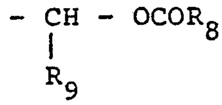

[worin $R_8$ einen Alkylrest oder den Cyclohexylrest und $R_9$ ein Wasserstoffatom oder einen Alkylrest bedeutet] oder einen Säureschutzrest bedeutet, wobei die obengenannten Alkylteile oder -reste gerade oder verzweigt sind und 1 bis 4 Kohlenstoffatome enthalten, das Produkt in der 3-Oxoäthyl-bicycloocten-2- oder -3-Form oder 3-Oxoäthyliden-bicyclooctan-Form vorliegt, falls n = 0, und in der 3-Oxoäthyl-bicyclooocten-2- oder 3-Oxoäthylidenbicyclooctan-Form vorliegt, falls n = 1, sowie die Gemische ihrer Isomeren.

2. Produkt gemäß Anspruch 1, dadurch gekennzeichnet, daß n, $R_2$, $R^a_5$, $R^b_5$ und $R^c_5$ wie in Anspruch 1 definiert sind, $R_4$ einen tert.-Butoxycarbonyl-, 2,2,2-Trichlor-äthoxycarbonyl-, Chloracetyl-, Trichloracetyl-, Trityl-, Benzyl-, Dibenzyl-, Benzyloxycarbonyl-, p-Nitrobenzyloxycarbonyl-, p-Methoxybenzyloxycarbonyl-, Formyl- oder Trifluoracetylrest bedeutet.

3. Produkt gemäß Anspruch 1 oder Anspruch 2, dadurch gekennzeichnet, daß die Säureschutzreste, die durch $R^c_5$ und/oder $R_2$ dargestellt sind, ausgewählt sind unter den Methoxymethyl-, tert.-Butyl-, Benzhydryl-, Benzyl-, Nitrobenzyl- oder p-Methoxybenzylresten.

4. Ein Cephalosporinderivat gemäß Anspruch 1, 2 oder 3, dadurch gekennzeichnet, daß n gleich 0 ist, die Symbole $R^a_5$ und $R^b_5$ Alkylreste bedeuten, die Symbole $R_2$ und $R^c_5$ Säureschutzreste bedeuten und das Symbol $R_4$ einen Aminschutzrest bedeutet.

5. Ein Cephalosporinderivat gemäß Anspruch 4, dadurch gekennzeichnet, daß es in 3-Oxoäthyl-bicyclooocten-2-Form vorliegt.

6. Ein Verfahren zur Herstellung eines Produkts gemäß Anspruch 1 oder gemäß den Ansprüchen 2 bis 5, wobei n gleich 0 ist und die anderen Symbole gemäß einem der Ansprüche 1 bis 5 definiert sind, dadurch gekennzeichnet, daß man ein Enamin der allgemeinen Formel

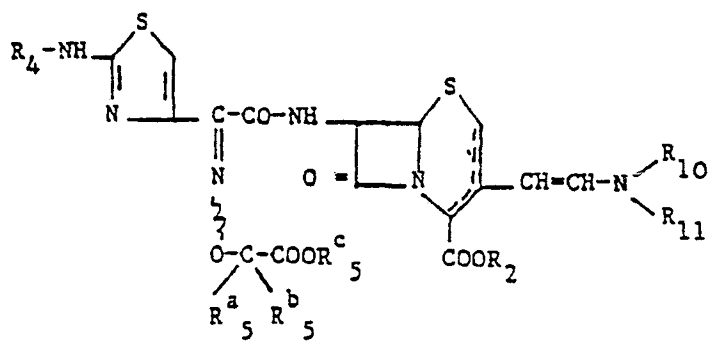

(oder ein Gemisch seiner Isomeren), worin $R_2$, $R_4$, $R^a_5$, $R^b_5$ und $R^c_5$ wie in Anspruch 1, 2 oder 3 definiert sind ($R^c_5$ anders als Wasserstoff ist) und $R_{10}$ und $R_{11}$, welche identisch oder verschieden sind, Alkylreste (gegebenenfalls substituiert durch einen Hydroxy-, Alkyloxy-, Amino-, Alkylamino- oder Dialkylaminorest) oder Phenyl bedeuten oder zusammen mit dem Stickstoffatom, an das sie gebunden sind, einen gesättigten Heterozyklus mit 5 oder 6 Kettengliedern bildet, der gegebenenfalls ein weiteres Heteroatom, ausgewählt unter Stickstoff, Sauerstoff oder Schwefel, enthält und gegebenenfalls durch einen Alkylrest substituiert ist, das in Bicyclooocten-2- oder -3-Form vorliegt und dessen Substituent am Kohlenstoffatom in 3-Stellung des Bicyclooctens die E- oder Z-Stereoisomerie aufweist, hydrolysiert, und daß man dann gegebenenfalls den Schutzrest $R^c_5$ entfernt und/oder gegebenenfalls die Isomeren des erhaltenen Produkts trennt.

7. Verfahren zur Herstellung eines Produkts gemäß Anspruch 1 oder gemäß einem der Ansprüche 2 bis 5, wobei n gleich 1 ist und die übrigen Symbole gemäß einem der Ansprüche 1 bis 5 definiert sind, dadurch gekennzeichnet, daß man ein Produkt gemäß Anspruch 1, wofür n = 0, oxydiert und dann gegebenenfalls die Isomeren des erhaltenen Produkts trennt.

**Anspruch** (für den Vertragsstaat AT)

Verfahren zur Herstellung eines Cephalosporins der allgemeinen Formel

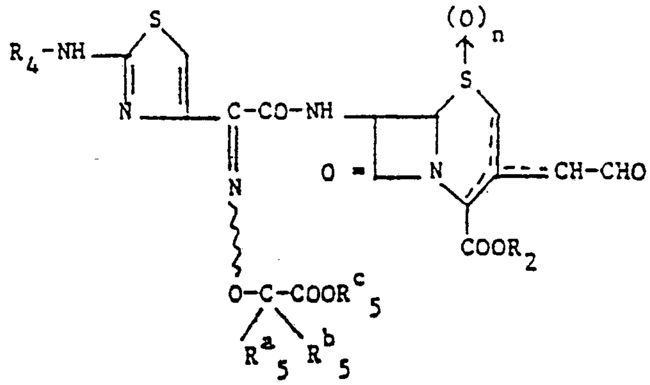

in syn- oder anti-Form, in welcher n gleich 0 oder 1 ist, die reste $R^a_5$ und $R^b_5$, die gleich oder voneinander verschieden sind, Wasserstoffatome oder Alkylreste darstellen oder zusammen einen Alkylenrest mit 2 oder 3 Kohlenstoffatomen bilden, $R^c_5$ ein Wasserstoffatom oder eine Säureschutzgruppe darstellt, $R_4$ eine Aminschutzgruppe darstellt und das Symbol $R_2$ einen leicht auf enzymatischem Wege abtrennbaren Rest der allgemeinen Formel

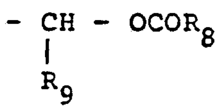

[worin $R_8$ einen Alkylrest oder den Cyclohexylrest darstellt und $R_9$ ein Wasserstoffatom oder einen Alkylrest bedeutet] oder eine Säureschutzgruppe darstellt, wobei die oben genannten Alkylteile oder -reste geradkettig oder verzweigt sind und 1 bis 4 Kohlenstoffatome aufweisen, die Verbindung in 3-Oxoäthyl-2- oder -3-bicycloocten-Form oder 3-Oxoäthyliden-bicyclooctan-Form vorliegt, wenn n = 0, und in 3-Oxoäthyl-2-bicycloocten-Form oder 3-Oxoäthyliden-bicyclooctan-Form vorliegt, wenn n = 1, sowie von den Mischungen ihrer Isomeren, dadurch gekennzeichnet, daß man zur Herstellung einer Verbindung, in deren Formel n gleich 0 ist, ein Enamin der allgemeinen Formel

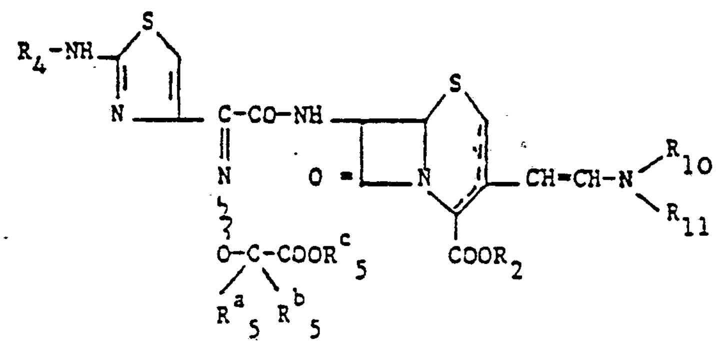

(oder eine Mischung seiner Isomeren), worin $R_2$, $R_4$, $R^a_5$, $R^b_5$ und $R^c_5$ die obige Bedeutung haben ($R^c_5$ ist von Wasserstoff verschieden) und $R_{10}$ und $R_{11}$, die gleich oder voneinander verschieden sind, Alkylreste (gegebenenfalls substituiert durch einen Hydroxy-, Alkyloxy-, Amino-, Alkylamino- oder Dialkylaminorest) oder Phenylreste darstellen oder zusammen mit dem Stickstoffatom, an das sie gebunden sind, einen gesättigten Heterocyclus mit 5 oder 6 Gliedern, der gegebenenfalls ein weiteres Heteroatom, ausgewählt aus Stickstoff, Sauerstoff oder Schwefel, enthält und gegebenenfalls durch einen Alkylrest substituiert ist, bilden, welches in 2- oder 3-Bicycloocten-Form vorliegt und dessen Substituent am in 3-Stellung des Bicyclooctens befindlichen Kohlenstoffatom E- oder Z-Stereoisomerie aufweist, hydrolysiert, dann gegebenenfalls den Rest $R^c_5$ entfernt und/oder gegebenenfalls die Isomeren der erhaltenen Verbindung trennt und dann zur Herstellung einer Verbindung, in deren Formel n gleich 1 ist, die entsprechende zuvor erhaltene Verbindung, worin n gleich 0 ist, oxidiert und dann gegebenenfalls die Isomeren der erhaltenen Verbindung trennt.